# EUROPEAN PATENT SPECIFICATION

(11) **EP 0 866 865 B1**
(45) Date of publication and mention of the grant of the patent: **06.02.2002**
(21) Application number: 96938213.4
(22) Date of filing: 14.11.1996
(51) Int. Cl.: C12N 15/52, C12N 9/00, A61K 31/70, C07H 21/02, C12N 15/11, C12Q 1/68

(54) **CHIMERIC OLIGOMERS HAVING AN RNA-CLEAVAGE ACTIVITY**
CHIMÄRE OLIGOMERE MIT EINER RNS-SPALTUNGSAKTIVITÄT
OLIGOMERES CHIMERIQUES POSSEDANT UNE ACTIVITE DE CLIVAGE DE L'ARN

(30) Priority: 14.11.1995 DE 19542404; 07.03.1996 US 612298
(43) Date of publication of application: 30.09.1998
(73) Proprietor: Vimrx Holdings, Ltd., Wilmington, DE 19808 (US)
(72) Inventor: LUDWIG, János, D-37085 Göttingen (DE); SPROAT, Brian, D-37139 Adelebsen (DE)
(74) Representative: Viering, Jentschura & Partner
(86) International application number: EP9605014
(87) International publication number: WO9718312

(56) References cited:
- WO-A-91/19789
- WO-A-92/07065
- WO-A-93/15187
- WO-A-94/10301
- WO-A-94/13789
- WO-A-95/11304
- NUCLEIC ACIDS RESEARCH, vol. 17, no. 4, 25 February 1989, pages 1371-1377, XP002026788 A.C. JEFFRIES ET AL.: "A catalytic 13-mer ribozyme" cited in the application
- THE EMBO JOURNAL , vol. 11, no. 4, April 1992, pages 1525-1530, XP000268587 P. STEINECKE ET AL.: "Expression of a chimeric ribozyme gene results in endonucleolytic cleavage of target mRNA and a concomitant reduction of gene expression in vivo"
- NUCLEIC ACIDS RESEARCH, vol. 20, no. 17, 1992, pages 4559-4565, XP000644533 N.R. TAYLOR ET AL.: "Chimeric DNA-RNA hammerhead ribozymes have enhanced in vitro catalytic efficiency and increased stability in vivo"
- THE EMBO JOURNAL, vol. 11, no. 5, May 1992, pages 1913-1919, XP000268586 G. PAOLELLA ET AL.: "Nuclease resistant ribozymes with high catalytic activity" cited in the application
- J. AM. CHEM. SOC., vol. 115, 1993, pages 8483-8484, XP000644539 F. BENSELER ET AL.: "Hammerhead-like molecules containing non-nucleoside linkers are active RNA catalysts" cited in the application
- JOURNAL OF CANCER RESEARCH AND CLINICAL ONCOLOGY 122 (4). 1996. 254-256. ISSN: 0171-5216, XP000618242 KOZU T ET AL: "Designing of chimeric DNA-RNA hammerhead ribozymes to be targeted against AML1-MTG8 mRNA."

## Description

The present invention concerns chimeric oligomers having an RNA-cleavage activity and the use thereof for the cleavage of RNA substrates in vitro and in vivo.

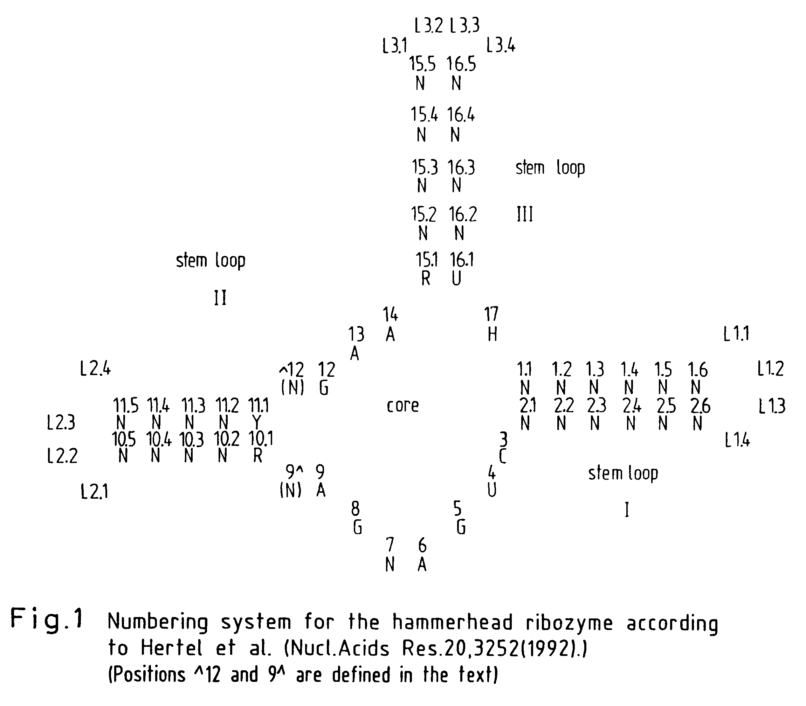

Hammerhead ribozymes are an example of catalytic RNA molecules which are able to recognize and cleave a given specific RNA substrate (Hotchins et al., Nucleic Acids Res. 14 (1986), 3627; Keese and Symons in: Viroids and viroid - like pathogens, J.J. Semanchik, Publ. (CRC-Press, Boca Raton, Florida, (1987), p. 1 - 47). The catalytic centre of hammerhead ribozymes contains three stems and can be formed by adjacent sequence regions of the RNA or also by regions which are separated from one another by many nucleotides. Fig 1 shows a diagram of such a catalytically active hammerhead structure. The stems have been denoted I, II and III. The nucleotides are numbered according to the standard nomenclature for hammerhead ribozymes (Hertel et al., Nucleic Acids Res. 20 (1992), 3252).

The consensus sequence of the catalytic core structure is described by Ruffner and Uhlenbeck (Nucleic Acids Res. 18 (1990), 6025-6029). Perriman et al. (Gene 113 (1992), 157 - 163) have meanwhile shown that this structure can also contain variations e.g. naturally occurring nucleotide insertions such as N^{9λ} and N^{λ12}, or modifications that can be tolerated such as R^{15.1}=G. Thus the positive strand of the satellite RNA of the tobacco ring-spot virus does not contain any of the two nucleotide insertions while the +RNA strand of the virusoid of the lucerne transient streak virus (vLTSV) contains a N^{9λ} = U insertion which can be mutated to C or G without loss of activity (Sheldon and Symons, Nucleic Acids Res. 17 (1989), 5679-5685). Furthermore in this special case N⁷ = A and R^{15.1} = A. On the other hand the minus strand of the carnation stunt associated viroid (- CarSV) is quite unusual since it contains both nucleotide insertions i.e. N^{λ12} = A and N^{9λ} = C (Hernandez et al., Nucleic Acids Res. 20 (1992), 6323-6329). In this viroid N⁷ = A and R^{15.1} = A. In addition this special hammerhead structure exhibits a very effective self-catalytic cleavage despite the more open central stem.

The possibilities of using hammerhead RNA enzymes range from the isolation of RNA restriction enzymes to the specific inactivation of the expression of genes in cells e.g. in animal, human or plant cells and in prokaryotes, yeasts and plasmodia. A particular biomedical interest is based on the fact that many diseases, including many forms of tumours, are related to the expression of specific genes. Inactivating such genes by cleaving the associated mRNA could represent a possible way to control and eventually treat such diseases. Moreover there is a great need to develop antiviral or antimicrobial pharmaceutical agents, the RNA enzymes possibly being such an agent, since viral expression can be blocked selectively by cleaving viral or microbial RNA molecules.

The most promising variants of pharmaceutical agents for the specific inactivation of the expression of genes are modified oligonucleotides which contain a block of deoxyribonucleotides in the middle region of the molecule (Giles et al., Nucleic Acids Res. 20 (1992), 763 - 770). These deoxyribonucleotides form a DNA-RNA hybrid with RNA the RNA strand of which is cleaved by cellular RNAse H. However, a disadvantage of these oligonucleotides for in vivo applications is their low specificity, since hybrid formation and thus cleavage can also take place at undesired positions on the RNA molecules. In addition the DNA sequences interact undesirably with cellular proteins.

Previous attempts to recombinantly express catalytically active RNA molecules of the cell by transfecting the cell with an appropriate gene have not proven to be very effective since a very high expression was necessary to inactivate specific RNA substrates. In addition the vector systems which are available now cannot generally be applied. Furthermore unmodified RNA enzymes cannot be administered directly due to the sensitivity of RNA to degradation by RNAses and their interactions with proteins.

Thus chemically modified active substances have to be administered in order to administer hammerhead ribozymes exogenously (cf. e.g. Heidenreich et al., J. Biol. Chem. 269 (1994), 2131-2138; Kiehntopf et al., EMBO J. 13 (1994), 4645-4652; Paolella et al., EMBO J. 11 (1992), 1913-1919 and Usman et al., Nucleic Acids Symp. Ser. 31 (1994), 163-164).

DE-OS 42 16 134 describes such chemically modified active substances based on synthetic catalytic oligonucleotide structures with a length of 35-40 nucleotides which are suitable for cleaving a nucleic acid target sequence and contain modified ribonucleotides that contain an optionally substituted alkyl, alkenyl or alkynyl group with 1 - 10 carbon atoms at the 2'-O atom of the ribose. Furthermore oligonucleotides are described which contain the above-mentioned modified nucleotide building blocks and which form a hammerhead structure. These oligonucleotides are able to cleave specific RNA substrates. Examples of oligonucleotides are described having an active centre which has a length of 14 nucleotides. This active centre contains several ribonucleotides that increase the sensitivity towards enzymes which cleave RNA. A further disadvantage is the length of the active centre which can often lead to unspecific hybridization.

WO 95/11304 describes RNA-cleaving nucleic acids with an active centre that is free of ribonucleotide building blocks but instead contains deoxyribonucleotides.However, the deoxyribonucleotides used in the active centre result in a very low RNA cleavage activity. Thus it was reported that a 13-mer deoxyribozyme of the "GAAA" type based on LTSV was not able to cleave a 41-mer oligoribonucleotide substrate while the corresponding 13-mer ribozyme exhibited an excellent catalytic activity (Jeffries and Symons, Nucleic Acids Res. 17 (1989), 1371-1377).

In addition the use of a larger number of deoxyribonucleotide building blocks in the hybridization arms or in the active centre leads to loss of specificity due to an activation of RNAse H since sequences which are related to the desired target sequence can also be cleaved. Moreover catalytic DNA oligomers are not particularly well suited for in vivo applications due to interactions with proteins.

The object of the present invention was therefore to provide chimeric oligomers that cleave RNA in which the disadvantages of the state of the art are at least partially eliminated. In particular it is intended to provide chimeric RNA-cleaving oligomers which at the same time have a high stability and a high effectivity and specificity as well as being suitable for in vivo applications.

This object is achieved by a chimeric oligomer having RNA cleavage activity which has a structure of formula (I):

5'-X-Z-Y-3' (I)

in which X and Y represent oligomeric sequences containing building blocks composed of nucleotides or/and nucleotide analogues and contribute to the formation of a specific hybridization of the oligomer with an RNA substrate,
in which X and Y each contain at least one building block that differs from deoxyribonucleotides and ribonucleotides
in which Z represents the active centre of the chimeric oligomer having a structure of formulae (IIIa), (IIIb), (IVa) or (IVb):

   5'-G¹²-A¹³A¹⁴R^{15.1}-3' (IIIa)

   5'-N^{λ12}G¹²A¹³A¹⁴R^{15.1}-3' (IIIb)

   5'-C³U⁴G⁵A⁶N⁷G⁸A⁹-3' (IVa)

   5'-C³U⁴G⁵A⁶N⁷G⁸A⁹N^{9λ}-3' (IVb)
in which the building blocks of the structures (IIIa), (IIIb), (IVa), (IVb) are selected from nucleotides or/and nucleotide analogues
in which the building blocks have a structure of formula (II): 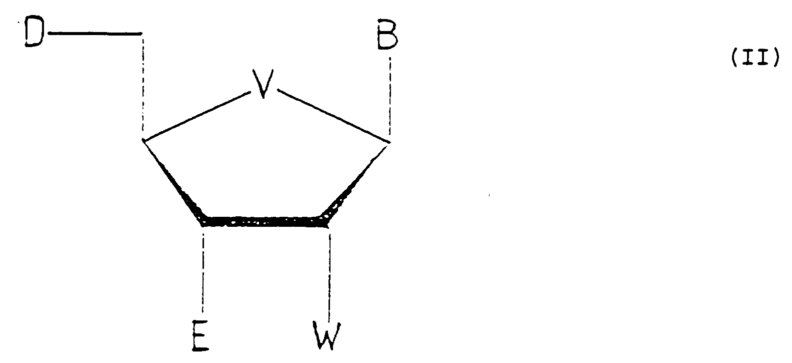
in which B denotes a residue which is a naturally occurring nucleobase or a functional equivalent thereof, in particular selected from the group comprising adenin-9-yl (A), cytosin-1-yl (C), guanin-9-yl (G), uracil-1-yl (U), uracil-5-yl (ψ), hypoxanthin-9-yl (I), thymin-1-yl (T), 5-methylcytosin-1-yl (5MeC), 2,6-diaminopurin-9-yl(aminoA), purin-9-yl (P), 7-deazaadenin-9-yl (c⁷A), 7-deazaguanin-9-yl (c⁷G), 5-propynylcytosin-1-yl (5-pC), 5-propynyluracil-1-yl(5-pU), isoguanin-9-yl, 2-aminopurin-9-yl, 6-methyluracil-1-yl, 4-thiouracil-1-yl, 2-pyrimidone-1-yl and quinazoline-2,4-dione-1-yl;
   V independently denotes an O, S, NH or CH₂ group W independently denotes an H or OH or a straight-chained or branched alkyl, alkoxy, alkenyl, alkenyloxy, alkynyl or alkynyloxy with 1 to 10 C atoms which can be optionally substituted by one or several halogen, cyano, amino, carboxy, ester, ether, carboxamide, hydroxyl or/and mercapto groups or is selected from -COOH, -CONH₂, -CONHR¹, -CONR¹R², -NH₂, -NHR¹, -NR¹R², -NHCOR¹, -SH, -SR¹, -F, -ONH₂, -ONHR¹, -ONR¹R², -NHOH, -NHOR¹, -NR²OH and NR²OR¹ in which R¹ and R² independently denote unsubstituted or substituted alkyl, alkenyl or alkynyl groups as defined above, D and E denote residues which together form a phosphodiester or phosphorothioate diester bond between adjacent nucleosides or analogues or together form an analogue of an internucleosidic bond,
in which the structures (IIIa) or (IIIb) do not contain a total of more than two deoxyribonucleotides and the structures (IVa) or (IVb) do not contain a total of more than four deoxyribonucleotides and
in which
   - G: in each case independently denotes a building block according to formula (II) in which B is guanin-9-yl or a functional equivalent thereof for that particular location in the hammerhead core structure, which is preferably selected from I, c⁷G and isoguanin-9-yl;
   - A: in each case independently denotes a building block according to formula (II) in which B is adenin-9-yl or a functional equivalent thereof for that particular location in the hammerhead core structure, which is preferably selected from aminoA, P, c⁷A and 2-aminopurin-9-yl;
   - U: in each case independently denotes a building block according to formula (II) in which B is uracil-1-yl or a functional equivalent thereof for that particular location in the hammerhead core structure, which is preferably selected from ψ, T, 5-pU, 6-methyluracil-1-yl,4-thiouracil-1-yl, 2-pyrimidone-1-yl and quinazoline-2,4-dione-1-yl;
   - C: in each case independently denotes a building block according to formula (II) in which B is cytosin-1-yl or a functional equivalent thereof for that particular location in the hammerhead core structure, which is preferably selected from 5MeC, 5-pC and 2-pyrimidone-1-yl;
   - R: in each case independently denotes a building block according to formula (II) in which B independently denotes G or A as defined above,
   - N: in each case independently denotes a building block according to formula (II).

The monomeric building blocks of sequences X and Y which flank the active centre are nucleotide or/and nucleotide analogues and are selected in such a way that they specifically hybridize with a given RNA substrate and, together with the active centre Z, form a structure, especially a hammerhead structure which specifically cleaves the RNA substrate.

The nucleotide building blocks can, on the one hand, be selected from ribonucleotides. However, the number of ribonucleotides should be as small as possible since the presence of ribonucleotide building blocks strongly reduces the in vivo stability of the oligomers. The flanking regions X and Y (and also the active centre Z) should in particular not contain any natural ribonucleotide building blocks at the positions taken up by pyrimidine nucleotides but rather modified building blocks.

The use of a larger number of deoxyribonucleotides for the flanking sequences X and Y is also less preferred since undesired interactions with proteins can occur or the formation of an RNAse H-sensitive DNA-RNA hybrid. Thus the flanking sequences each preferably contain at most 1 and particularly preferably no ribonucleotide at all and a consecutive sequence of a maximum of 3 deoxyribonucleotides and especially no more than 2 deoxyribonucleotides.

The building blocks of the flanking sequences X and Y are preferably selected from nucleotides or/and nucleotide analogues e.g. from building blocks having the structure (II) which are also used for the active centre Z. Preferred modified building blocks are those in which W is in each case independently selected from optionally substituted alkyl, alkoxy, alkenyl, alkenyloxy, alkynyl or alkynyloxy residues with 1 - 10 C atoms. Particularly preferred substituents are for example methyl, ethyl, propyl, allyl, methoxy, ethoxy, propoxy, allyloxy, hydroxyethyloxy and methoxyethyloxy. However, other modified building blocks can also be used such as nucleotide analogues which contain amino groups, substituted amino groups, halogen groups or sulfhydryl groups at the 2' position of pentose.

The internucleosidic linkage between two nucleoside building blocks can be achieved by phosphodiester bonds or by modified phospho bonds such as by phosphorothioate groups or other bonds such as for example those described in DE-OS 42 16 134.

In addition the flanking regions X and Y can also contain nucleotide analogues such as peptidic nucleic acids (see for example Nielsen et al., Science 254 (1991), 1497-1500 and Dueholm et al., J. Org. Chem. 59 (1994), 5767-5773). In this case the linkage of individual building blocks can for example be achieved by acid amide bonds. The linkage of flanking regions X and Y which are based on peptidic nucleic acids with an active centre Z based on nucleotide building blocks can either be achieved by suitable linkers (see e.g. Petersen et al., BioMed. Chem. Lett. 5 (1995), 1119-1121) or directly (Bergmann et al., Tetrahedron Lett. 36 (1995), 6823-6826.

The flanking regions X and Y preferably contain independently of each other 3 - 40 and particularly preferably 5 - 10 nucleotide or nucleotide analogue building blocks.

The building blocks of the flanking regions X and Y contain nucleobases or analogues which can hybridize with bases that occur naturally in RNA molecules. The nucleobases are preferably selected from naturally occurring bases (adenine, guanine, cytosine, thymine and uracil) as well as analogues thereof e.g. 2,6-diaminopurine, hypoxanthine, 5-methylcytosine, pseudouracil, 5-propynyluracil, 5-propynylcytosine etc. which enable a specific binding to the target RNA.

A firm binding to the RNA substrate is preferred in the regions X and Y so that the following nucleobases are particularly preferred as building blocks: 2,6-diaminopurineinstead of adenine; thymine or 5-propynyluracil instead of uracil; 5-methylcytosine or 5-propynylcytosine instead of cytosine. In addition 2-amino-2'-O-alkyladenosines are preferred (Lamm et al., Nucleic Acids Res. 19 (1991), 3193-3198). Furthermore aromatic systems can be linked to positions 4 and 5 of uracil, such as B = phenoxazine by which means dramatic improvements are obtained in the stability of the double-strand (Lin et al., J. Am. Chem. Soc. 117 (1995), 3873-3874).

The 3' end of the oligomer according to the invention can additionally be protected against degradation by exonucleases for example by using a modified nucleotide building block that is also modified at the 3' position of the ribose sugar e.g. by an optionally substituted alkyl, alkoxy, alkenyl, alkenyloxy, alkynyl or alkynyloxy group as defined above. An additional possibility of further stabilizing oligomers according to the invention at the 3' end against degradation by exonucleases is a 3'-3'-linked dinucleotide structure or/and the presence of two modified phospho bonds such as two phosphorothioate bonds.

Moreover the chimeric oligomer structure according to the invention can be linked to a prosthetic group in order to improve its cellular uptake or/and to enable a specific cellular localization. Examples of such prosthetic groups are polyamino acids (e.g. polylysine), lipids, hormones or peptides. These prosthetic groups are usually linked via the 3' or 5' end of the oligomer either directly or by means of suitable linkers (e.g. linkers based on 6-aminohexanol or 6-mercapto-hexanol etc.). These linkers are commercially available and techniques suitable for linking prosthetic groups to the oligomer are known to a person skilled in the art.

The increase in the rate of hybridization is particularly important for the biological activity of the oligomers according to the invention since in this way it is possible to achieve a high activity at low concentrations. This is particularly important for short-lived RNA substrates or RNA substrates that occur less often. A substantial acceleration of the hybridization can for example be achieved by coupling positively charged peptides, which for example contain several lysine residues, to the end of an oligonucleotide (Corey, J. Am. Chem. Soc. 117 (1995), 9373-9374). A chimeric oligomer according to the invention can be simply modified in this manner using the linkers described above. Alternatively the rate of hybridization can also be increased by incorporation of building blocks which contain sperminyl residues (Schmid and Behr, Tetrahedron Lett. 36 (1995), 1447 - 1450). Such modifications of the chimeric oligomers also improve the ability to bind to RNA substrates having secondary structures.

The active centre Z of the chimeric oligomer according to the invention contains nucleotide building blocks which are modified and optionally a small number of natural nucleotides i.e. ribonucleotides or/and deoxyribonucleotides.

The nucleotide building blocks in the region Z are preferably selected from compounds which can only hybridize weakly with ribonucleotides such as modified nucleotides that contain an optionally substituted alkyl, alkenyl or alkynyl group with preferably 1-5 C atoms at the 2'-C atom of ribose. In addition substituents are also preferred which are selected from optionally substituted alkoxy, alkenyloxy or alkynyloxy groups with 1-5 C atoms. Particularly preferred nucleobases in the region Z are A or P, U, C and G or I.

According to a first embodiment of the present invention the active centre Z of the chimeric oligomer contains a structure of formulae (IIIa) or (IIIb). Such an oligomer can cleave an RNA substrate which contains a structure of formula (Va) or (Vb) :

5'-U^{16.1}H¹⁷-S-C³U⁴G⁵A⁶N⁷G⁸A⁹R^{10.1}-3' (Va)

5'-U^{16.1}H¹⁷-S-C³U⁴G⁵A⁶N⁷G⁸A⁹N^{9λ}R^{10.1}-3' (Vb)

in which N, H, R, S, G, A, U and C represent ribonucleotides and
N denotes G, A, U or C;
H denotes A, C or U;
R denotes A or G and
S is an RNA sequence capable of forming a hairpin structure with a length of preferably 6 - 60 and particularly preferably of 6 - 20 bases.

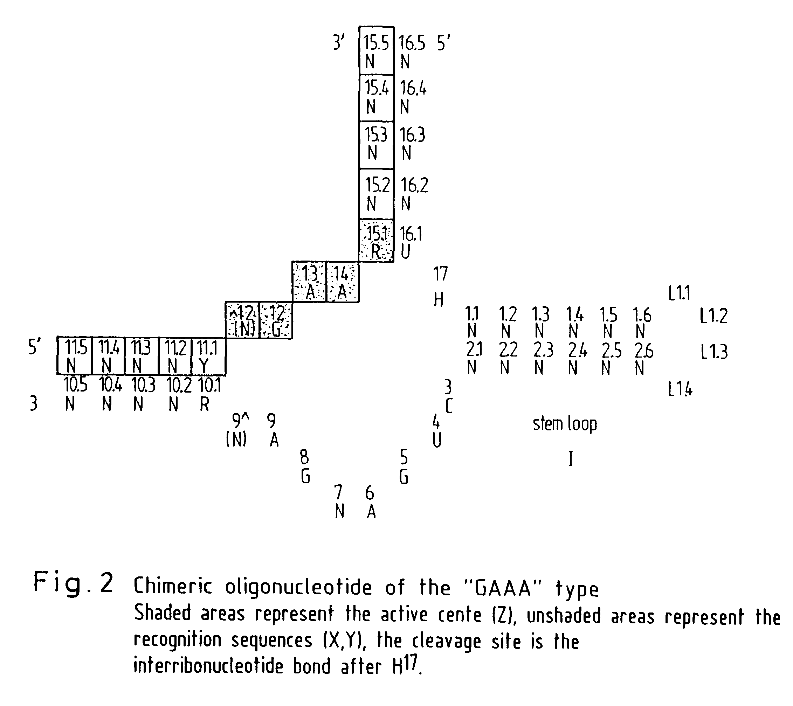

An oligomer with an active centre (IIIa) or (IIIb) can form the hammerhead structure shown in Fig. 2 with an RNA substrate containing a structure of formulae (Va) or (Vb) in which the nucleotides are numbered according to the standard nomenclature. The active centre Z (IIIa) or (IIIb) preferably contains no more than four and particularly preferably no more than two ribonucleotides and most preferably no more than one ribonucleotide.

Particularly good results were obtained for oligomers in which G12 is a deoxyribonucleotide, a ribonucleotide or a building block in which W is a C₁-C₄ alkyl, alkenyl, alkoxy or alkenyloxy group optionally substituted by OH and in particular a (2-hydroxyethyloxy) group. A¹³, A¹⁴ cr/and R^{15.1} are preferably selected from building blocks in which W is preferably a C₁-C₄ alkyl, alkenyl, alkoxy or alkenyloxy group optionally substituted by OH and particularly preferably a methoxy, (2-hydroxy-ethyloxy) and allyloxy group. In addition it was also possible to synthesize an oligomer which does not contain any ribonucleotide and any deoxyribonucleotidein the active centre Z. This oligomer contains a 2'-O-(2-hydroxyethyl)guanosine at position G¹² and a 2'-O-(2-hydroxyethyl)adenosine at each of the positions A¹³, A¹⁴ and R^{15.1}.

According to a second embodiment of the present invention the active centre of the oligomer has a structure of formulae (IVa) or (IVb) and exhibits cleavage activity for an RNA substrate that contains a structure of formula (VIa) or (VIb) :

5'-Y^{11.1}G¹²A¹³A¹⁴R^{15.1}-S-U^{16.1}H¹⁷-3' (VIa)

5'-Y^{11.1}N^{λ12}G¹²A¹³A¹⁴R^{15.1}-S-U^{16.1}H¹⁷-3' (VIb)

in which N, H, Y, R, S, G, A, U and C represent ribonucleotides and
N denotes G, A, U or C
H denotes A, C or U
R denotes A or G
Y denotes C or U and
S is an RNA sequence capable of forming a hairpin structure with a length of preferably 6 - 60 and particularly preferably of 6 - 20 bases.

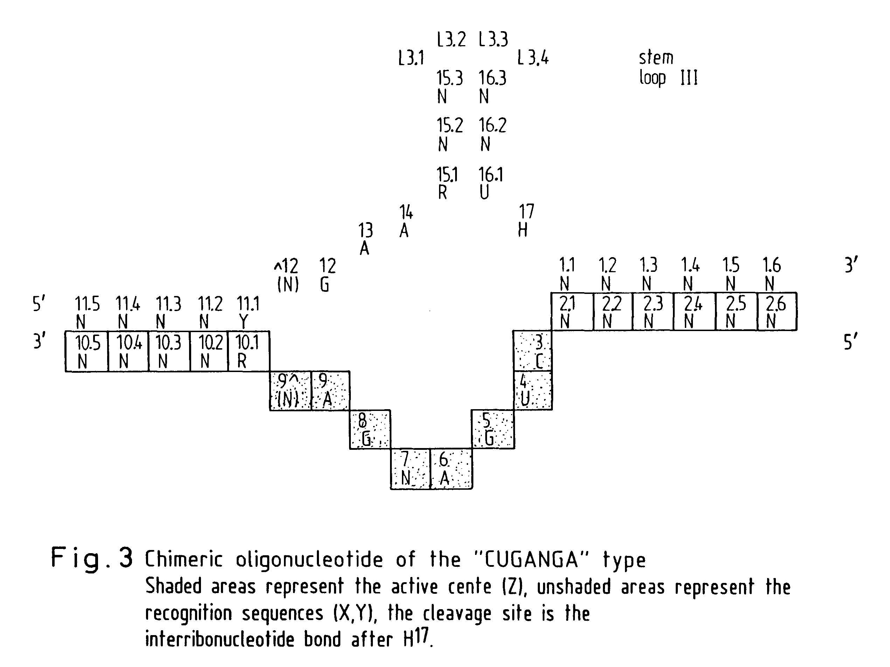

An oligomer with an active centre (IVa) or (IVb) forms the hammerhead structure shown in Fig. 3 with an RNA substrate containing a structure of formulae (VIa) or (VIb) in which the nucleotides are numbered according to the standard nomenclature. The active centre Z preferably contains no pyrimidine ribonucleotides. Z preferably contains no more than six and particularly preferably no more than three ribonucleotides.
If the active centre Z contains ribonucleotides, one or several building blocks at positions G⁵, A⁶, N⁷ (if N⁷ is a purine), G⁸, A⁹ and N^{9λ} (if N^{9λ} is present and is a purine) are thus preferably ribonucleotides. The building blocks at positions C³, U⁴ N⁷ (if N⁷ is a pyrimidine) and N^{9λ} (if N^{9λ} is present and is a pyrimidine) are preferably nucleotide analogue building blocks in particular such building blocks in which W is an optionally OH-substituted C₁-C₄ alkyl, alkenyl, alkoxy or alkenoxy group or an NH₂ group. Building blocks are especially preferred which can only hybridize very weakly with ribonucleotides in the substrate such as those nucleotide analogues which contain an optionally substituted alkyl, alkenyl or alkynyl group according to formula (II), e.g. an allyl group, at the 2' C atom of the ribose. However, 2'-O-nucleotide analogues are on the other hand also preferred. Specific examples of suitable substituents are methoxy, (2-hydroxyethyloxy), allyl, allyloxy and NH₂. In addition N⁷ and N^{9λ}, if they are present, are selected in such a way that a minimum of internal structures can form by internal interactions. The preferred nucleobases in the region Z are A or P, U, C and G or I.

In the following examples are given for suitable building blocks within the active centre of the chimeric oligomers according to the invention based on results of standard hammerhead ribozymes: The following building blocks are preferred for chimeric oligomers of the "CUGANGA" type having an active centre of formula (IVa) or (IVb):
Position C³ B=C, V=O, W=allyloxy; B=C, V=O, W=allyl
Position U⁴ B=U, V=O, W=allyloxy; B=U, V=O, W=allyl
Position G⁵ B=G, V=O, W=amino
Position A⁶ B=A, V=O, W=H; B=P, V=O, W=OH
Position N⁷ B=U, V=O, W=allyloxy; B=C, V=O, W=allyl
Position G⁸ B=G, V=O, W=amino; B=I, V=O, W=OH
Position R⁹ B=A, V=O, W=H; B=P, V=O, W=OH; B=A, V=O, W=allyloxy

The following building blocks are preferred for chimeric oligomers of the "GAAR" type having an active centre of formula (IIIa) or (IIIb):
Position G¹² B=G, V=O, W=H; B=c⁷G, V=O, W=OH; B=G, V=O, W=2-hydroxyethyloxy
Position A¹³ B=A, V=O, W=allyloxy; B=A, V=O, W=2-hydroxyethyloxy;
Position A¹⁴ B=A, V=O, W=allyloxy; B=P, V=O, W=OH; B=A, V=O, W=2-hydroxyethyloxy
Position R^{15.1} B=A, V=O, W=OH; B=A, V=O, W=2-hydroxyethyloxy

Computer algorithms can be used to identify RNA substrates in sequence databases for oligomeric chimeras according to the invention. For oligomeric chimeras having an active centre GAAR such an algorithm is for example as follows (numbering according to Fig. 2):
- i :: find all C³ UGANGA(N)R sequences in a given mRNA.
- ii:: identify N^{2.1} and find potential N^{1.1}-N^{2.1} base pairs (in which N^{1.1} must be part of an N^{16.2}, U^{16.1}, H¹⁷, N^{1.1} sequence) in a region of C³ positioned ca. 30 nucleotides in the 3' direction.
- iii:: calculate stem stabilities for stems which terminate at the above-mentioned N^{1.1}-N^{2.1} base pairs.
- iv:: sort according to stem stability.

For chimeric oligomers with the active centre CUGANGA, RNA substrates can for example be determined as follows: (numbering according to Fig. 3):
- i:: find all Y(N)GAAR^{15.1} sequences in a given mRNA.
- ii:: find potential R^{15.1}-U^{16.1} base pairs in a region ca. 30 nucleotides in the 5' direction of R^{15.1}, in which N¹⁷ is not allowed to be G.
- iii:: calculate the stem stabilities for stems that terminate at the above-mentioned base pair
- iv:: sort according to stem stability.

A program based on these algorithms enables a very efficient search in databases or individual sequences. As a result, in addition to a suitable RNA target sequence, one obtains the sequence of the chimeric oligonucleotide which is necessary to cleave this target sequence.

In this connection it is important to also take into consideration motifs containing incomplete base pairs in the region of the stem structures I and III since several incomplete base pairs (mismatches) can be tolerated in these sections.

Using a computer program based on the above-mentioned algorithm it was possible to identify about 12,000 suitable motifs in human sequences (4.1 x 10⁷ nucleotides) and about 4,000 motifs in viral sequences (2 x 10⁷ nucleotides) for oligomers with the active centre "GAAR". The number of motifs for oligomers with the active centre CUGANGA is ca. 50,000 motifs in human sequences if R^{15.1} = A.

The minimum stability requirements for a hammerhead structure are three base pairs in stem I and three base pairs in stem III. Although all motifs which fulfil these minimum requirements can be cleaved, the cleavage efficiency depends on the interaction of the actual recognition sequences and on conserved nucleotides in the active centre.

Of the oligomers according to the invention the chimeric oligonucleotides containing GAAR seem to be less sensitive to interferences by undesired interactions and are therefore preferred.

RNA substrates of chimeric oligomers with an active centre which has a structure of formulae (IIIa) or (IIIb) are preferably human cellular transcripts and transcripts of human viruses. The RNA substrate is particularly preferably selected from the group comprising human interleukin-2 mRNA, human ICAM-1 mRNA, human TGF-β mRNA, human tissue factor pre-mRNA, human protein kinase C-α mRNA, human factor KBF1 mRNA, human 5-lipoxygenase mRNA, human interleukin-8 RNA, human interleukin-5 RNA, human interleukin-1 receptor mRNA, human interleukin-1-α mRNA, human 12-lipoxygenase mRNA, the transcript of the human cytomegalovirus DNA polymerase gene and a transcript of the human papilloma virus type 8.

Particularly preferred cleavage motifs for oligomers of the "GAAR" type are located at the following positions of these RNA substrates (the name of the respective sequence in the EMBL Nucleotide Sequence Database 43rd Edition is given in brackets).
- human interleukin-2-mRNA (HSIL2R) with C³ at position 490 and a cleavage after the sequence AUU;
- human ICAM-1 (intercellular adhesion molecule-1)mRNA (HSI-CAM01) with C³ at position 1933 and a cleavage after the sequence AUU;
- human TGF-β (transforming growth factor β)mRNA (HSTGFB1) with C³ at position 1313 and a cleavage after the sequence CUC;
- human tissue factor pre-mRNA (HSTFPB) with C³ at position 6781 and a cleavage after the sequence GUU or with C³ at position 8831 and a cleavage after the sequence AUC or CUU;
- human PKC-α (protein kinase C-α)mRNA (HSPKCA1) with C³ at position 823 and a cleavage after the sequence UUC or UUU;
- human factor KBF-1 mRNA (HSNFKB34) with C³ at position 2619 and a cleavage after the sequence UUA;
- human 5-lipoxygenase mRNA (HSLOX5A) with C³ at position 296 and a cleavage after the sequence GUA;
- human interleukin-8 RNA (HSIL8A) with C³ at position 4978 and a cleavage after the sequence AUA;
- human interleukin-5 RNA (HSIL5) with C³ at position 1896 and a cleavage after the sequence AUU or UUU;
- human interleukin-1 receptor mRNA (HSIL1RA) with C³ at position 1485 and a cleavage after the sequence AUC;
- human interleukin-1-α mRNA (HSIL1ALPH) with C³ at position 1272 and a cleavage after the sequence CUU;
- human 12-lipoxygenase mRNA (HSLIPXYG) with C³ at position 940 and a cleavage after the sequence AUA;
- transcript of the human cytomegalovirus DNA polymerase gene (HEHS5POL) with C³ at position 3919 and a cleavage after the sequence UUA;
- transcript of the human papilloma virus type 8 (PAPPPH8C) with C³ at position 3221 and a cleavage after the sequence CUU.

A particularly preferred example of a cleavage site in a human mRNA is interleukin-2 mRNA. The cleavage site has the nucleotide sequence shown in SEQ ID NO. 1. An oligomer of the "GAAR" type having the nucleotide sequence shown in SEQ ID NO. 2 is also able to efficiently cleave an appropriate RNA substrate. This oligomer according to the invention can also cleave a 1.6 kb long IL-2 transcript.

Human ICAM-1 mRNA is a further particularly preferred example of an RNA substrate which can be cleaved by the chimeric oligomers of the "GAAR" type. The region which is sensitive for cleavage has the nucleotide sequence shown in SEQ ID NO. 3. An RNA substrate with this sequence can also be efficiently cleaved by an oligomer having the nucleotide sequence shown in SEQ ID NO. 4.

Human PKC-α mRNA is a further particularly preferred example of an RNA substrate, which can be cleaved by a chimeric oligomer of the "GAAR" type. The region which is sensitive to cleavage has the nucleotide sequence shown in SEQ ID NO. 9. This substrate is efficiently cleaved by an oligomer having the nucleotide sequence shown in SEQ ID. N0. 10.

Human interleukin-1α mRNA is a further particularly preferred example of an RNA substrate which can be cleaved by a chimeric oligomer of the "GAAR" type. The region which is sensitive to cleavage has the nucleotide sequence shown in SEQ ID NO. 11. This substrate is efficiently cleaved by an oligomer having the nucleotide sequence shown in SEQ ID NO. 12.

Preferred RNA substrates for oligomers according to the invention of the "CUGANGA" type are also human cellular transcripts and transcripts of human viruses. The RNA substrate is particularly preferably selected from the group comprising human interleukin-6 mRNA, human multiple drug resistance (MDR-1) mRNA, human monocyte chemotactic protein RNA, human macrophage scavenger receptor type II mRNA, human macrophage scavenger receptor type I mRNA, human macrophage inflammatory protein-1α mRNA, human p53 RNA, human jun-B mRNA, human c-jun RNA, human interferon γ type II mRNA, human hepatocyte growth factor mRNA, human HER2 mRNA, human Alzheimer's disease amyloid mRNA, human interleukin-1 mRNA, human interleukin-1 receptor mRNA, human 3-hydroxy-3-methylglutaryl coenzyme A reductase RNA, human angiotensinogen mRNA, human angiotensin-convertingenzyme mRNA, human acyl coenzyme A: cholesterol acyltransferase mRNA, human PDGF receptor mRNA, human TNF receptor mRNA, human TGF β mRNA, human NF-kappa B p65 subunit mRNA, human c-myc RNA, human 12-lipoxygenase mRNA, human interleukin-4 RNA, human interleukin-10 mRNA, human basic FGF mRNA, human EGF receptor mRNA, human c-myb mRNA, human c-fos RNA, human bcl-2 mRNA, human bcl-1 mRNA, human ICAM-1 mRNA, a transcript of human papilloma virus type 11 and transcripts of human papilloma virus type 16 and type 18.

Specific examples of suitable substrates are as follows:
- human monocyte chemotactic protein RNA (HSMCHEMP) with Y^{11.1} at position 2183 and a cleavage after the sequence GUU;
- human macrophage scavenger receptor type II RNA (HSPHSR2) with Y^{11.1} at position 833 and a cleavage after the sequence CUC;
- human macrophage scavenger receptor type I mRNA (HSPHSR1) with Y^{11.1} at position 813 and a cleavage after the sequence CUC;
- human macrophage inflammatory protein 1-α mRNA (HSRANTES) with A^{11.1} at position 629 and a cleavage after the sequence CUC;
- human p53 RNA (HSP53G) with Y^{11.1} at position 19145 and a cleavage after the sequence GUC;
- human jun-B mRNA (HSJUNB) with Y^{11.1} at position 357 and a cleavage after the sequence GUC;
- human C-jun RNA (HSJUNA) with Y^{11.1} at position 3066 and a cleavage after the sequence CUC;
- human interferon-γ type II mRNA (HSIFNGAMM) with Y^{11.1} at position 720 and a cleavage after the sequence GUU;
- human hepatocyte growth factor mRNA (HSHGF) with Y^{11.1} at position 1197 and a cleavage after the sequence GUC or with Y^{11.1} at position 1603 and a cleavage after the sequence GUA;
- human HER2 (tyrosine kinase receptor) mRNA (HSHER2A) with Y^{11.1} at position 2318 and a cleavage after the sequence CUU;
- human Alzheimer's disease amyloid mRNA (HSAMY) with Y^{11.1} at position 427 and a cleavage after the sequence AUC or with Y^{11.1} at position 1288 and a cleavage after the sequence CUA;
- human interleukin-1 mRNA (HSIL1) with Y^{11.1} at position 113 and a cleavage after the sequence AUU;
- human interleukin-1 receptor (IL-1R) mRNA (HSIL1RA) with Y^{11.1} at position 84 and a cleavage after the sequence CUC;
- human 3-hydroxy-3-methylglutarylcoenzyme A reductase mRNA (HSHMGCOB) with Y^{11.1} at position 257 and a cleavage after the sequence CUC;
- human angiotensinogen mRNA (HSANG) with Y^{11.1} at position 2012 and cleavage after the sequence UUC;
- human angiotensin-convertingenzyme mRNA (HSACE) with Y^{11.1} at position 1818 and a cleavage after the sequence GUA;
- human acyl coenzyme A: cholesterol acyltransferase mRNA (HSACYLCOA) with Y^{11.1} at position 374 and a cleavage after the sequence CUA or with Y^{11.1} at position 830 and a cleavage after the sequence GUA;
- human PDGF receptor mRNA (HSPDGFRA) with Y^{11.1} at position 1513 and a cleavage after the sequence CUA;
- human tumour necrosis factor receptor mRNA (HSTNFRB) with Y^{11.1} at position 502 and a cleavage after the sequence CUU or UUC;
- human TGF β mRNA (HSTGFBC) with Y^{11.1} at position 545 and a cleavage after the sequence AUC or with Y^{11.1} at position 2428 and a cleavage after the sequence CUC;
- human NF kappa B p65 subunit mRNA (HSNFKB65A) with Y^{11.1} at position 937 and a cleavage after the sequence UUC or with Y^{11.1} at position 2195 and a cleavage after the sequence CUC;
- human c-myc RNA (HSMYCC) with Y^{11.1} at position 4336 and a cleavage after the sequence CUU or with Y^{11.1} at position 3799 and a cleavage after the sequence CUA;
- human c-myc mRNA (HSMYC1) with Y^{11.1} at position 484 and a cleavage after the sequence CUU;
- human MDR-1 mRNA (HSMDR1) with Y^{11.1} at position 1513 and a cleavage after the sequence AUA;
- human 12-lipoxygenase mRNA (HSLIPXYG) with Y^{11.1} at position 566 and a cleavage after the sequence CUC;
- human interleukin-6 mRNA (HSIL6CSF) with Y^{11.1} at position 13 and a cleavage after the sequence CUA or with Y^{11.1} at position 902 and a cleavage after the sequence GUA;
- human interleukin-4 RNA (HSIL4A) with Y^{11.1} at position 2500 and a cleavage after the sequence GUC;
- human interleukin-10 mRNA (HSIL10) with Y^{11.1} at position 1125 and a cleavage after the sequence CUU;
- human basic FGF mRNA (HSGFBF) with Y^{11.1} at position 260 and a cleavage after the sequence CUA;
- human EGF receptor mRNA (HSEGFPRE) with Y^{11.1} at position 4778 and a cleavage after the sequence AUA or with Y^{11.1} at position 5508 and a cleavage after the sequence UUC;
- human c-myb mRNA (HSCMYBLA) with Y^{11.1} at position 448 and a cleavage after the sequence GUU;
- human c-fos RNA (HSCFOS) with Y^{11.1} at position 1065 and a cleavage after the sequence GUU or with Y^{11.1} at position 3349 and a cleavage after the sequence UUU;
- human bcl-2 mRNA (HSBSL2C) with Y^{11.1} at position 5753 and a cleavage after the sequence UUA;
- human bcl-1 mRNA (HSBCL1) with Y^{11.1} at position 3725 and a cleavage after the sequence GUC;
- human ICAM-1 mRNA (HSICAM01) with Y^{11.1} at position 1998 and a cleavage after the sequence GUA;
- a transcript of the human papilloma virus type 11 (PAPPPH11) with Y^{11.1} at position 2941 and a cleavage after the sequence AUU;
- a transcript of the human papilloma virus type 16 (PA16) with Y^{11.1} at position 37 and a cleavage after the sequence GUU;
- a transcript of the human papilloma virus type 16 (PA16) with Y^{11.1} at position 1126 and a cleavage after the sequence UUA;
- a transcript of the human papilloma virus type 16 (PA16) with Y^{11.1} at position 1322 and a cleavage after the sequence GUA;
- a transcript of the human papilloma virus type 16 (PA16) with Y^{11.1} at position 1982 and a cleavage after the sequence AUU;
- a transcript of the human papilloma virus type 16 (PA16) with Y^{11.1} at position 6440 and a cleavage after the sequence UUA;
- a transcript of the human papilloma virus type 16 L1 protein gene (PAPHPU111) with Y^{11.1} at position 99 and a cleavage after the sequence GUA;
- a transcript of the human papilloma virus type 18 (PAPPPH18) with Y^{11.1} at position 896 and a cleavage after the sequence AUA;
- transcript of the human papilloma virus type 18 E6 protein gene (PARHPVE6) with Y^{11.1} at position 172 and a cleavage after the sequence AUA.

A particularly preferred RNA substrate for a chimeric oligomer of the "CUGANGA" type is human interleukin-6 mRNA. The region which is sensitive to cleavage has the nucleotide sequence shown in SEQ ID NO. 5. An oligomer having the nucleotide sequence shown in SEQ ID NO. 6 can efficiently cleave such a substrate.

Yet a further particularly preferred example of an RNA substrate which can be cleaved by an oligomer of the "CUGANGA" type is human MDR-1 mRNA. The region which is sensitive to cleavage has the nucleotide sequence shown in SEQ ID NO. 7. An oligomer having the nucleotide sequence shown in SEQ ID No. 8 can efficiently cleave such an RNA substrate. A 1.6 kb long MDR-1 transcript can also be cleaved by this oligomer.

A further particularly preferred example of an RNA substrate which can be cleaved by an oligomer of the "CUGANGA" type is human c-jun RNA. The region which is sensitive to cleavage is shown in SEQ ID NO. 13. An oligomer having the nucleotide sequence shown in SEQ ID NO. 14 can cleave such a substrate.

A further particularly preferred example of an RNA substrate which can be cleaved by an oligomer of the "CUGANGA" type is human IL-1 receptor mRNA. The region which is sensitive to cleavage is shown in SEQ ID NO. 15. An oligomer having the nucleotide sequence shown in SEQ ID NO. 16 can efficiently cleave such a substrate.

A further particularly preferred example of an RNA substrate which can be cleaved by an oligomer of the "CUGANGA" type is human angiotensinogen mRNA. The region which is sensitive to cleavage is shown in SEQ ID NO. 17. An oligomer having the nucleotide sequence shown in SEQ ID NO. 18 can efficiently cleave such a substrate.

The chimeric oligomers according to the invention have significant advantages compared to the structures of the state of the art. Thus the shortest ribozymes that have been previously used have a minimum length of 15+N+M nucleotides, the active centre being 15 nucleotides long and N and M being the length of the recognition sequences (Benseler et al., J. Am. Chem. Soc. 115 (1993), 8483-8484), and moreover they contain ribonucleotides in at least five positions of the catalytic centre (Paolella et al., EMBO J. 11 (1992), 1913-1919 and Yang et al., Biochemistry 31 (1992), 5005-5009).

In contrast the oligomers according to the invention only contain 4+N+M or 5+N+M nucleotides (in the case of the "GAAR" type) or 7+N+M or 8+N+M nucleotides (in the case of the "CUGANGA" type) in which N and M are preferably numbers in the range of 5-10. Furthermore they can contain a significantly smaller number of natural nucleotides without loss of activity. Due to the reduced length and reduced number of ribonucleotides there are significant advantages with regard to the synthesis of the molecules as well as with regard to the stability in vivo. Thus for example the chimeric oligomer described in example 2 of the present application of the "GAAR" type has a half-life in active 10 % human serum of several days. The in vivo stability can be additionally increased by a further reduction in the number of ribonucleotides.

In addition the oligomers according to the invention have a very high in vivo activity since the RNA cleavage is promoted by protein factors that are present in the nucleus or cytoplasm of the cell. Examples of such protein factors which can increase the activity of hammerhead ribozymes are for example the nucleocapsid protein NCp7 of HIVl (Müller et al., J. Mol. Biol. 242 (1994), 422-429) and the heterogeneous nuclear ribonucleoprotein A1 (Heidenreich et al., Nucleic Acids Res. 23 (1995), 2223-2228). Thus long RNA transcripts can be cleaved highly efficiently within the cell by the oligomers according to the invention.

Yet a further advantage of the chimeric oligomers according to the invention is the statistically rare occurrence of cleavage motifs of formulae (Va) or (Vb), or (VIa) or (VIb) (approximately one motif/5000 bp) This taken together with the individually selected recognition sequences results in the fact that statistically only the target RNA within the entire human RNA pool is cleaved by the respective chimeric oligomer, whereas only an unproductive binding but no cleavage occurs at other potential binding sites. In addition the oligomers according to the invention do not activate RNAse H due to their low content of deoxyribonucleotides and thus do not cause any unspecific cleavages.

A further surprising advantage compared to ribozymes of the state of the art is that in the structures according to the invention the RNA substrate forms a large part of the hammerhead structure by which means the dependence of the cleavage activity on the Mg²⁺ concentration is positively influenced.

Yet a further object of the present invention is a pharmaceutical composition that contains one or several chimeric oligomers as the active substance and optionally pharmaceutically acceptable auxiliary substances, additives and carriers. This pharmaceutical composition is excellently suitable for the production of an agent to specifically inactivate the expression of genes in eukaryotes, prokaryotes and viruses especially of human genes such as tumour genes or viral genes or RNA molecules in a cell. Further areas of application are the inactivation of the expression of plant genes or insect genes. Thus the oligomers according to the invention can be used as a drug for humans and animals as well as a pesticide for plants.

For therapeutic applications the active substance is preferably administered at a concentration of 0.01 to 10,000 µg/kg body weight, particularly preferably of 0.1 to 1000 µg/kg body weight. The administration can for example be carried out by injection, inhalation (e.g. as an aerosol), as a spray, orally (e.g. as tablets, capsules, coated tablets etc.), topically or rectally (e.g. as suppositories).

The present invention provides a process for the specific inactivation of the expression of genes in which an active concentration of a chimeric oligomer is taken up into a cell (Lyngstadaas et al., EMBO J. 14 (1995) 5224-5229) so that the oligomer specifically cleaves a predetermined RNA molecule which is present in the cell, the cleavage preferably occurring catalytically. This process can be carried out in vitro on cell cultures as well as in vivo on living organisms (prokaryotes or eukaryotes such as humans, animals or plants).

Yet a further object of the present invention is the use of the chimeric oligomers as RNA restriction enzymes as well as a reagent kit for the restriction cleavage of RNA molecules which contains a chimeric oligomer and suitable buffer substances. In this case the oligomer and the buffer substances can be present in the form of solutions, suspensions or solids such as powders or lyophilisates. The reagents can be present together, separated from one another or optionally also on a suitable carrier. The oligomers according to the invention can also be used as a diagnostic agent or to identify the function of unknown genes.

This invention provides a method of alleviating psoriasis in a subject comprising administering to the subject an effective amount of the chimeric oligomers of this invention capable of cleaving RNAs encoding IL-2, or ICAM-1. This invention also provides a method of alleviating common cold in a subject comprising administering to the subject an effective amount of the chimeric oligomers of this invention capable of cleaving RNAs encoding ICAM-1. This invention further provides a method of alleviating transplant rejection in a subject comprising administering to the subject an effective amount of the chimeric oligomers of this invention capable of cleaving RNAs encoding ICAM-1. Moreover, this invention provides a method of alleviating Kaposi's sarcoma in a subject comprising administering to the subject an effective amount of chimeric oligomers of this invention capable of cleaving RNAs encoding IL-6.

This invention also provides a method of treating human cancer in a subject comprising administering to the subject an effective amount of the chimeric oligomers of this invention capable of cleaving RNAs encoding PKC-α. The important role played by PKC-α in cancer is described in Dean et al. (Cancer Research 56 (1996), 3499-3507) and in McKay et al. (Nucleic Acids Research 24 (1996), 411-417) and in references contained therein.

Finally, this invention provides a method of treating hypertension in a human subject comprising administering to the subject an effective amount of the chimeric oligomers of this invention capable of cleaving RNAs encoding angiotensinogen.

All documents referred to in this application are incorporated in their entirety herein by reference.

The invention is additionally elucidated by the following figures and sequence protocols. They show:
- Fig. 1: the schematic representation of a hammerhead structure and the corresponding nomenclature;
- Fig. 2: a hammerhead structure with an oligomer of the "GAAR" type according to the invention;
- Fig. 3: a hammerhead structure with an oligomer of the "CUGANGA" type according to the invention;
- Fig. 4a: a hammerhead structure which is formed from an interleukin-2 mRNA substrate and an oligomer according to the invention, wherein small letters denote ribonucleotides;
- Fig. 4b: the reaction of the structures shown in Fig. 4a;
- Fig. 5a: a hammerhead structure which is formed from an ICAM-1 mRNA substrate and an oligomer according to the invention, wherein small letters denote ribonucleotides;
- Fig. 5b: the reaction of the structures shown in Fig. 5a;
- Fig. 5c: the reaction of the structures shown in Fig. 5a when the chimeric oligomer has 2'-allyloxy-nucleotideanalogues in the flanking regions and U is replaced by 2'-allyloxy-thymidine;
- Fig. 6a: a hammerhead structure which is formed from an interleukin-6 mRNA substrate and an oligomer according to the invention, wherein small letters denote ribonucleotides;
- Fig. 6b: the reaction of the structures shown in Fig. 6a;
- Fig. 6c: the reaction of the structures shown in Fig. 6a whereby the substrate is 5'-fluorescentlylabelled and a variety of chimeric oligomers were tested with different analogues in the active centre "CUGAUGA";
- Fig. 7a: a hammerhead structure which is formed from an MDR-1 mRNA substrate and an oligomer according to the invention, wherein small letters denote ribonucleotides;
- Fig. 7b: the reaction of the structures shown in Fig. 7a;
- Fig. 8a: a hammerhead structure which is formed from a PKC-α mRNA substrate and an oligomer according to the invention, wherein small letters denote ribonucleotides;
- Fig. 8b: the reaction of the structures shown in Fig. 8a;
- Fig. 9a: a hammerhead structure which is formed from an interleukin-1α mRNA substrate and an oligomer according to the invention, wherein small letters denote ribonucleotides;
- Fig. 9b: the reaction of the structures shown in Fig. 9a;
- Fig. 10a: a hammerhead structure which is formed from a c-jun RNA substrate and an oligomer according to the invention, wherein small letters denote ribonucleotides;
- Fig. 10b: the reaction of the structures shown in Fig. 10a;
- Fig. 11a: a hammerhead structure which is formed from an interleukin-1 receptor mRNA substrate and an oligomer according to the invention, wherein small letters denote ribonucleotides;
- Fig. 11b: the reaction of the structures shown in Fig. 11a;
- Fig. 12a: a hammerhead structure which is formed from an angiotensinogen mRNA substrate and an oligomer according to the invention, wherein small letters denote ribonucleotides;
- Fig. 12b: the reaction of the structures shown in Fig. 12a;
- SEQ ID NO. 1: shows the nucleotide sequence of the interleukin-2 RNA substrate shown in Fig. 4a;
- SEQ ID NO. 2: shows the sequence of the RNA-cleaving oligomer shown in Fig. 4a;
- SEQ ID NO. 3: shows the nucleotide sequence of the ICAM-1 RNA substrate shown in Fig. 5a;
- SEQ ID NO. 4: shows the nucleotide sequence of the RNA-cleaving oligomer shown in Fig. 5a;
- SEQ ID NO. 5: shows the nucleotide sequence of the interleukin-6 RNA substrate shown in Fig. 6a;
- SEQ ID NO. 6: shows the nucleotide sequence of the RNA-cleaving oligomer shown in Fig. 6a;
- SEQ ID NO. 7: shows the nucleotide sequence of the MDR-1 RNA substrate shown in Fig. 7a;
- SEQ ID NO. 8: shows the nucleotide sequence of the RNA-cleaving substrate shown in Fig. 7a;
- SEQ ID NO. 9: shows the nucleotide sequence of the PKC-α RNA substrate shown in Fig. 8a;
- SEQ ID NO. 10: shows the nucleotide sequence of the RNA-cleaving oligomer shown in Fig. 8a;
- SEQ ID NO. 11: shows the nucleotide sequence of the interleukin-1α RNA substrate shown in Fig. 9a;
- SEQ ID NO. 12: shows the nucleotide sequence of the RNA-cleaving oligomer shown in Fig. 9a;
- SEQ ID NO. 13: shows the nucleotide sequence of the c-jun RNA substrate shown in Fig. 10a;
- SEQ ID NO. 14: shows the nucleotide sequence of the RNA-cleaving oligomer shown in Fig. 10a;
- SEQ ID NO. 15: shows the nucleotide sequence of the interleukin-1 receptor RNA substrate shown in Fig. 11a;
- SEQ ID NO. 16: shows the nucleotide sequence of the RNA-cleaving oligomer shown in Fig. 11a;
- SEQ ID NO. 17: shows the nucleotide sequence of the angiotensinogen RNA substrate shown in Fig. 12a and
- SEQ ID NO. 18: shows the nucleotide sequence of the RNA-cleaving oligomer shown in Fig. 12a.

In the sequences stated in SEQ ID NO. 1-18 the T at the end of the nucleotide is in each case coupled via its 3' position to the 3' position of the penultimate nucleotide (iT).

The invention is elucidated further by the following examples.

### Example 1

### Cleavage of an interleukin-2 RNA substrate

An interleukin-2 RNA substrate having the nucleotide sequence shown in SEQ ID NO. 1 was produced by chemical solid phase synthesis. Furthermore a chimeric oligomer was synthesized having the nucleotide sequence shown in SEQ ID NO. 2. Synthesis and removal of protecting groups was carried out in the case of the RNA substrate as well as of the chimeric oligomer by means of solid phase techniques (Wincott et al., Nucleic Acids Res. 23 (1995), 2677-2684). Purification was by means of anion-exchange HPLC (Sproat et al., Nucleosides and Nucleotides 14 (1995), 255-273). It was synthesized on an inverse thymidine support (Ortigao et al., Antisense Research and Development 2 (1992), 129-146) based either on aminopropyl-modifiedcontrolled pore glass or preferably aminomethylpolystyrene (McCollum and Andrus, Tetrahedron Letters 32 (1991), 4069-4072). Suitable protected 2'-methoxynucleoside-3'-O-phosphoramidites were synthesized as described by Sproat and Lamond in Oligonucleotides and Analogs - A practical approach, F. Eckstein, publ. (IRL-Press, Oxford, UK, (1991), 49-86). The 2'-allyloxy-nucleotide monomers for the solid phase synthesis were also synthesized by standard procedures (Sproat in: Methods in Molecular Biology, Vol. 20: Protocols for Oligonucleotides and Analogs - Synthesis and Properties, S. Agrawal, publ./ Humana Press, Totowa, New Jersey, (1993), 115-141)).

The sequences of the chimeric oligomer outside of the active centre are composed of nucleotide analogue building blocks which are modified with a methoxy group at the 2'-C atom of the ribose. In the active centre "GAAA" of the oligonucleotide G¹² at position 1 is a ribonucleotide, the residues A¹³ and A¹⁴ at positions 2 and 3 are 2'-methoxy-modifiednucleotides and A^{15.1} at position 4 is a ribonucleotide.

400 pmol interleukin-2 substrate was reacted with 400 pmol of the oligomer in the presence of 10 mmol/l MgCl₂, 50 mmol/l Tris HCl, 1 mmol/l EDTA at pH 7.5 and 37°C in a reaction volume of 100 µl. Aliquots were taken at certain times and admixed with excess EDTA to prevent further cleavage. The oligomer mixture present at the different times was then separated by electrophoresis on a 20 % polyacrylamide gel in the presence of 7 M urea and made visible by staining with "stains-all".

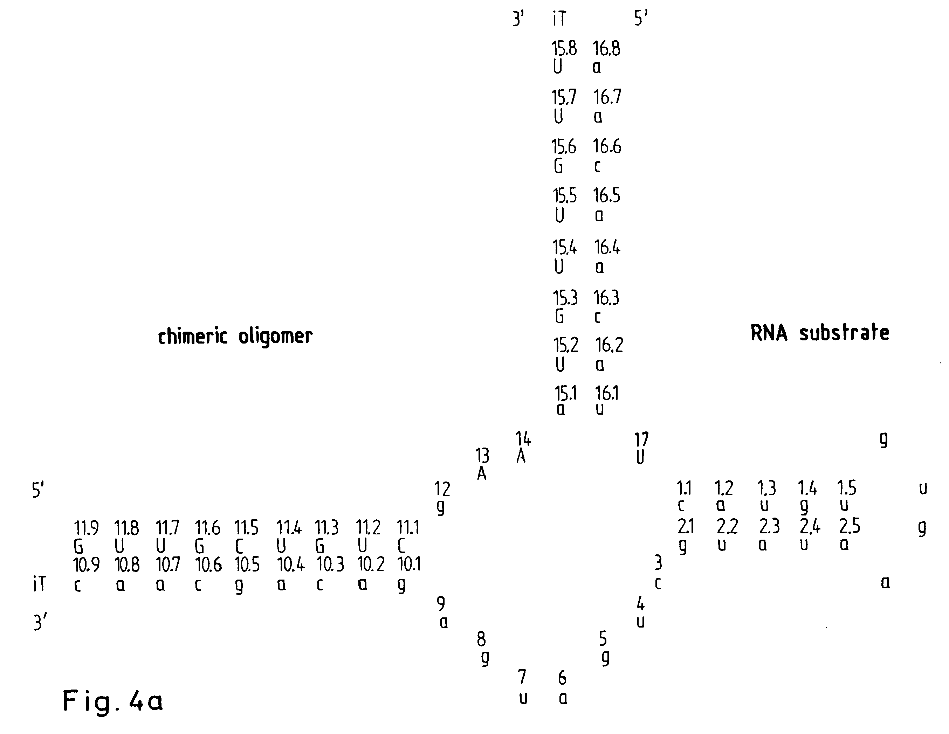

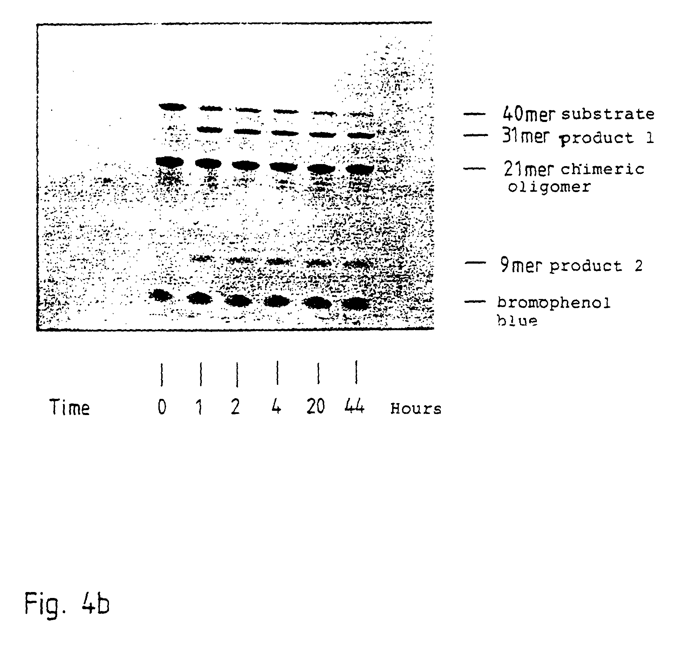

The result of this reaction is shown in Fig. 4b. This shows that the band (40-mer) allocated to the substrate disappears as the incubation period increases and the intensity of the bands of the cleavage products (31-mer and 9-mer) increases.

Similar results to those shown in Fig. 4b were obtained with an oligomer that contains a deoxyguanosine at position G¹² instead of a guanosine. The following two sequences of the active centre Z also led to an effective cleavage of the 40-mer RNA substrate but with a slightly lower cleavage rate:
(i) position G¹²: guanosine; positions A¹³ and A¹⁴: 2'-O-methyladenosine; position A^{15.1}: 2'-O-(2-hydroxyethyl)-adenosine;
(ii) position G¹²: guanosine; positions A¹³, A¹⁴ and A^{15.1}: 2'-O-(2-hydroxyethyl)-adenosine.

### Example 2

### Cleavage of an ICAM-1 RNA substrate

An ICAM-1 RNA substrate having the nucleotide sequence shown in SEQ ID NO. 3 and an RNA-cleaving oligomer having the nucleotide sequence shown in SEQ ID NO. 4 were synthesized in an analogous manner to that described in example 1. The nucleotide building blocks used for the oligomer were also the same as those described in example 1 except that A¹³ and A¹⁴ were ribonucleotides.

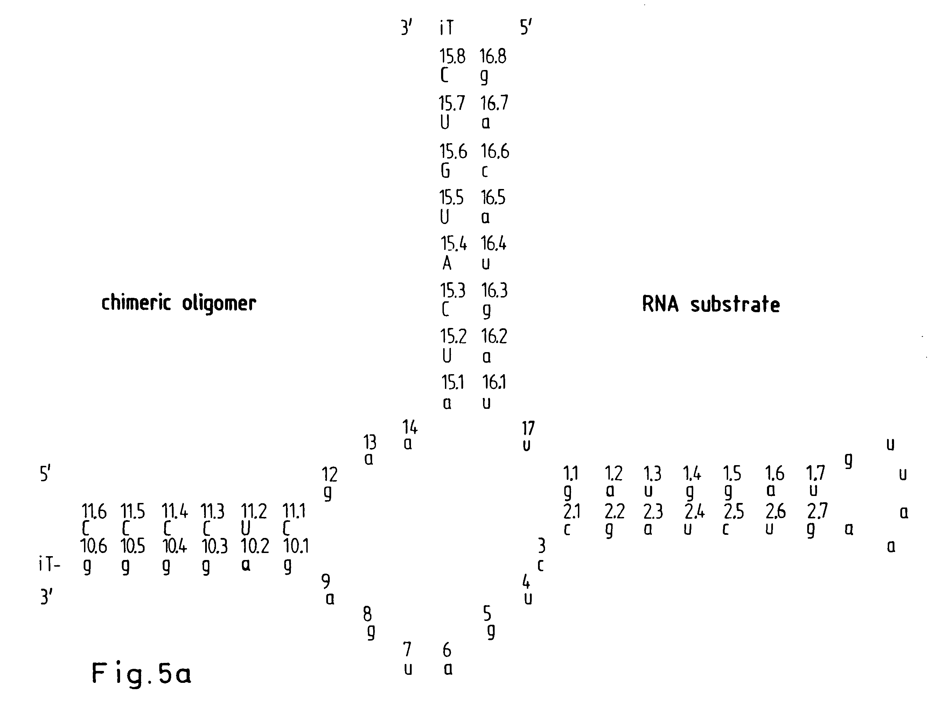

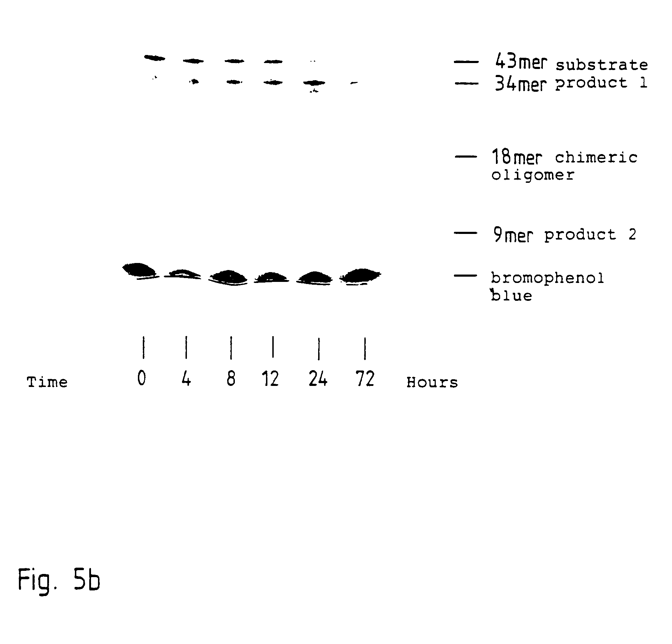

The result of the reaction is shown in Fig. 5b. The reaction conditions were as described in example 1 except that 1 nmol substrate and 100 pmol chimeric oligomer were used. It can be seen that the band of the substrate (43-mer) disappears as the incubation period increases while the intensity of the bands of the products (34-mer and 9-mer) increases.

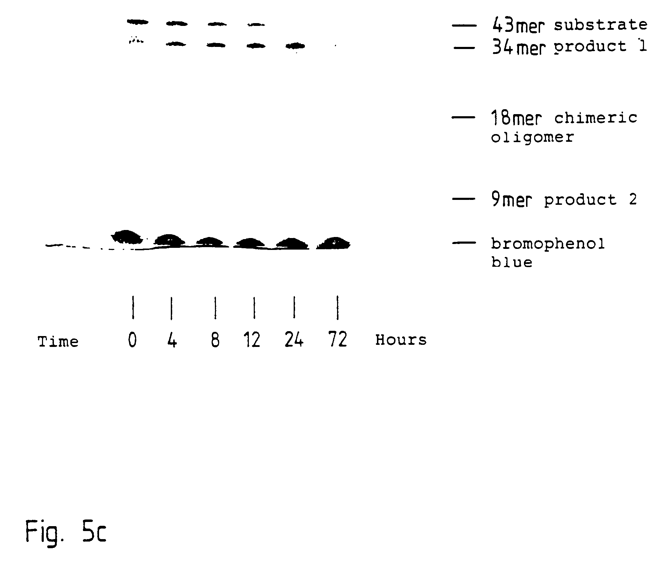

A chimeric oligomer was synthesized in an analogous manner which contained four ribonucleotides in the active centre Z and 2'-allyloxy-nucleotidesin the flanking regions X and Y. 2'-Allyloxythymidine was used instead of 2'-O-allyl-uridine at the positions in X and Y labelled U. The results of the cleavage of the 43-mer RNA substrate by this oligomer are shown in Fig. 5c.

Oligomers based on 2'-O-allyloxy-nucleotides are strongly preferred over oligomers based on 2'-methoxy-nucleotides (Iribarren et al., Proc. Natl. Acad. Sci. USA 87 (1990), 7747-7751) especially for therapeutic applications due to their greatly reduced unspecific binding.

### Example 3

### Cleavage of an interleukin-6 RNA substrate

An interleukin-6 RNA substrate having the nucleotide sequence shown in SEQ ID NO. 5 and a chimeric oligomer having the nucleotide sequence shown in SEQ ID NO. 6 were synthesized in an analogous manner to that described in example 1. 2'-Methoxymodified nucleotides were used in sequences outside the active centre to produce the oligomer. Ribonucleotides were used for the active centre "CUGAUGA".

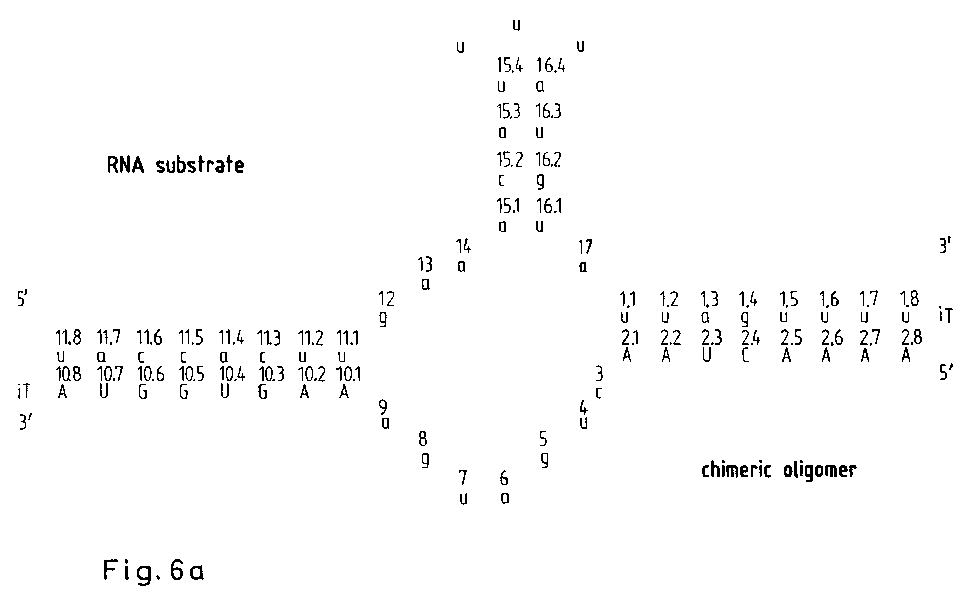

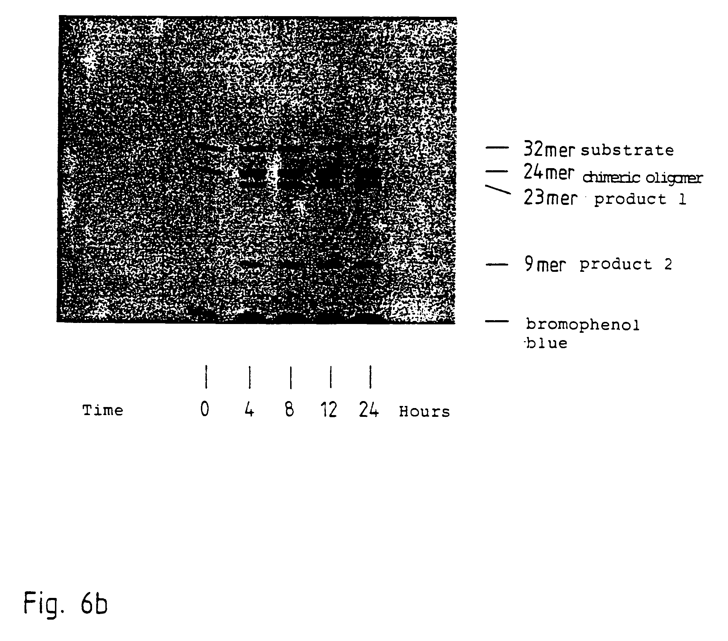

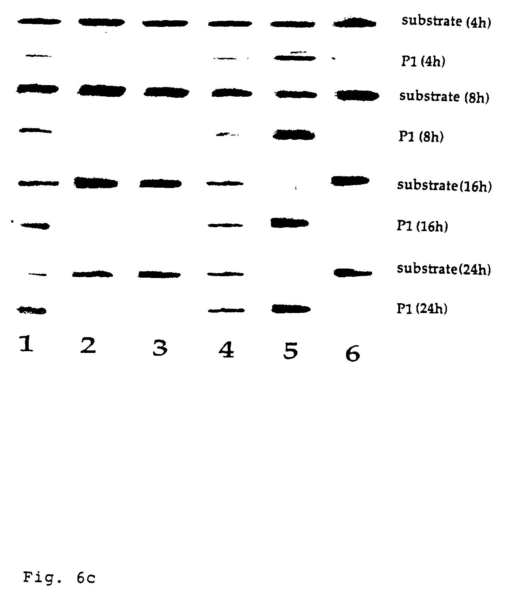

The reaction was carried out as described in example 1. The result is shown in Fig. 6b. The intensity of the bands of the substrate (32-mer) decreases as the incubation period increases and the intensity of the bands of the cleavage products (23-mer and 9-mer) increases.

Further reactions were performed as follows: A 5'-fluorescein labelled substrate having the nucleotide sequence shown in SEQ ID NO. 5 was synthesized using a commercially available fluorescein phosphoramidite monomer, and subjected to cleavage reactions as described above, however using a variety of chimeric oligomers each having the nucleotide sequence shown in SEQ ID N0. 6, all bearing 2'-allyloxy modified nucleotides in regions X and Y but different analogues in the active centre "CUGANGA". The reactions were carried out as described in example 1, except that the chimeric oligomer concentration was 10 µmol/l and the initial substrate concentration was 2 µmol/l. The time course of the reaction is shown in Fig. 6c using fluorescence detection, so only the full length fluorescein labelled 32mer substrate and the 5'-fluorescein labelled 23mer product 1 are detected. The lanes comprised the following:

Lane 1: Substrate plus chimeric oligomer with 2'-C-allyl-2'-deoxycytidine at position C³, 2'-amino-2'-deoxyuridine at positions U⁴ and U⁷ and ribonucleotides at positions G⁵, A⁶, G⁸, and A⁹ in the active centre "CUGANGA".

Lane 2: Substrate plus chimeric oligomer with 2'-C-allyl-2'-deoxycytidine at position C³, 2'-C-allyl-2'-deoxyuridine at positions U⁴ and U⁷ and ribonucleotides at positions G⁵, A⁶, G⁸, and A⁹ in the active centre "CUGANGA".

Lane 3: Substrate plus chimeric oligomer with 2'-allyloxy-2'-deoxycytidine at position C³, 2'-C-allyl-2'-deoxyuridine at positions U⁴ and U⁷ and ribonucleotides at positions G⁵, A⁶, G⁸, and A⁹ in the active centre "CUGANGA".

Lane 4: Substrate plus chimeric oligomer with 2'-allyloxy-2'-deoxycytidine at position C³, 2'-amino-2'-deoxyuridine at positions U⁴ and U⁷ and ribonucleotides at positions G⁵, A⁶, G⁸, and A⁹ in the active centre "CUGANGA".

Lane 5: Substrate plus chimeric oligomer containing all ribonucleotides in the active centre "CUGANGA".

Lane 6: Substrate only.

The chimeric oligomer used in lane 5 is clearly the most efficient, however, since it contains pyrimidine ribonucleotides in the active centre "CUGANGA" it is less suitable for in vivo use. The chimeric oligomers used in lanes 1 and 4 are somewhat less active, however they contain no pyrimidine ribonucleotides and are thus very well suitable for cell culture and in vivo work.

### Example 4

### Cleavage of a MDR-1 RNA substrate

A MDR-1 RNA substrate having the nucleotide sequence shown in SEQ ID NO. 7 and an RNA-cleaving oligomer having the nucleotide sequence shown in SEQ ID NO. 8 were synthesized in an analogous manner to that described in example 1. The oligomer was synthesized using the nucleotide building blocks stated in example 3.

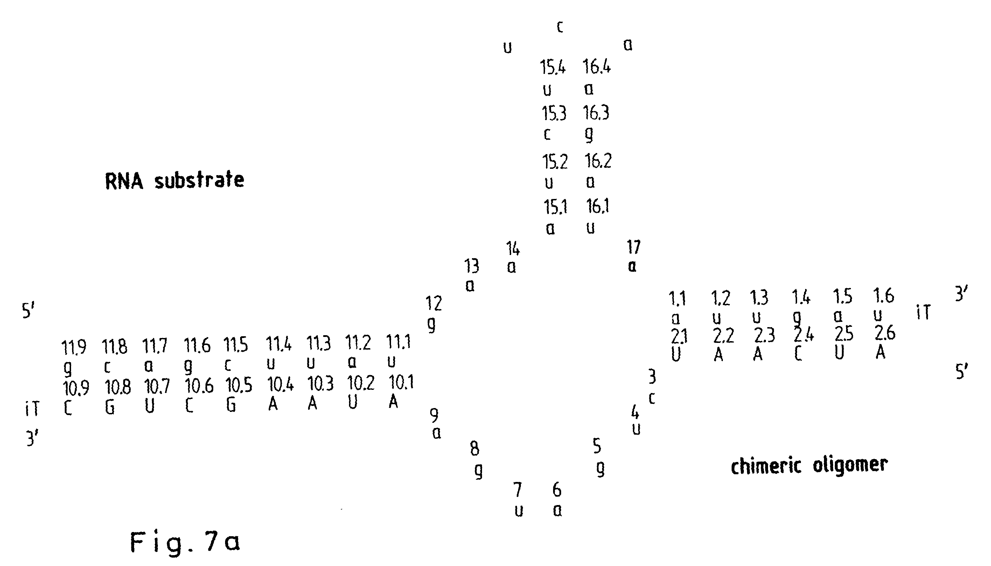

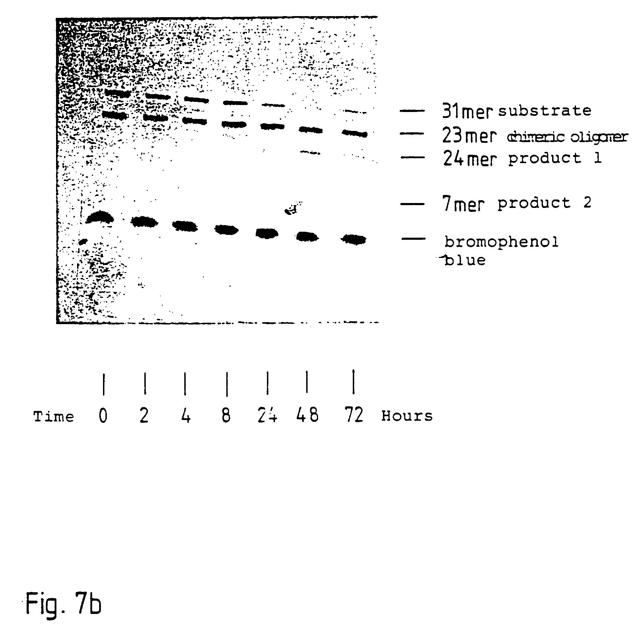

The reaction was carried out as described in example 1.
The result is shown in Fig. 7b.

### Example 5

Synthesis, removal of protecting groups and purification of a chimeric oligomer of the "GAAA" type for cleaving an RNA motif on human ICAM-1 RNA

The chimeric oligomer 5'-CCCCT*CgaaaT*CAT*GT*C-iT, in which capital letters denote 2'-allyloxy-nucleotides (T* is 2'-allyloxythymidine), small letters denote ribonucleotides and -iT denotes a 3'-3'-phosphodiester bond to deoxythymidine, was synthesized according to the "trityl-off" technique by a solid phase-β-cyanoethyl phosphoroamidite technique (Sinha et al., Nucleic Acids Res. 12 (1984), 4539-4557) on a 1 µmol scale using an inverse thymidine support (Ortigao et al., Supra), protected 2'-allyloxy-nucleotidemonomers (Sproat in Methods in Molecular Biology, Supra) and commercially available 2'-O-tert.-butyldimethylsilyl-protected ribonucleotide monomers using a longer coupling period of 15 min. At the end of the synthesis the oligomer was cleaved in the synthesis apparatus from the controlled pore glass or polystyrene suppcrt during a period of 2 h using 2 ml 33 % aqueous ammonia/ethanol (3:1 v/v). The solution was then kept at 60°C for 6-8 h in a sealed reaction vessel in order to remove all base-labile protecting groups. The reaction vessel was cooled and the solution was evaporated to dryness.

The residue was taken up in 300 µl ethanol/water (1:1 v/v) and transferred to a 1.5 ml reaction vessel. Subsequently the solution was again lyophilized.

Silyl protecting groups were removed by resuspending the residue in 200 µl dry triethylamine/triethyl-amine· 3 HF/N-methyl-2-pyrrolidinone (3:4:6 v/v/v) and incubating for 2 h at 60°C (Wincott et al., Supra). The chimeric oligomer which had been completely freed of protecting groups was then precipitated by addition of 20 µl 3 M aqueous sterile sodium acetate and subsequently of 1 ml 1-butanol. The reaction vessel was kept at -20°C for 15 minutes and the precipitate was collected by centrifugation for 10 min. The supernatant was carefully removed by decanting, the pellet was taken up in 300 µl 80 % ethanol and the resulting solution was lyophilized.

The crude product was firstly analyzed and then purified for 30 min in four aliquots by anion-exchange HPLC on a Dionex Nucleopak PA-100 column (9 x 250 mm) at 55°C using a lithium perchlorate gradient of 70-230 mM in water/acetonitrile (9:1 v/v) containing 25 mM Tris pH 7.0. The main peak was collected and the chimeric oligomer was precipitated by addition of 4 volumes of 1-propanol or 2-propanol (Sproat et al., (1995) Supra). The precipitate was collected by centrifugation, washed and then dried in a vacuum. The yield of purified product was 2.8 mg.

### Example 6

### Cleavage of a PKC-α RNA substrate

A PKC-α RNA substrate having the nucleotide sequence shown in SEQ ID NO. 9 and an RNA-cleaving oligomer having the nucleotide sequence shown in SEQ ID NO. 10 were synthesized in an analogous manner to that described in example 1. The oligomer was synthesized with 2'-allyloxy-modified nucleotides in the sequences outside the active centre, and with ribonucleotides in the active centre "GAAA".

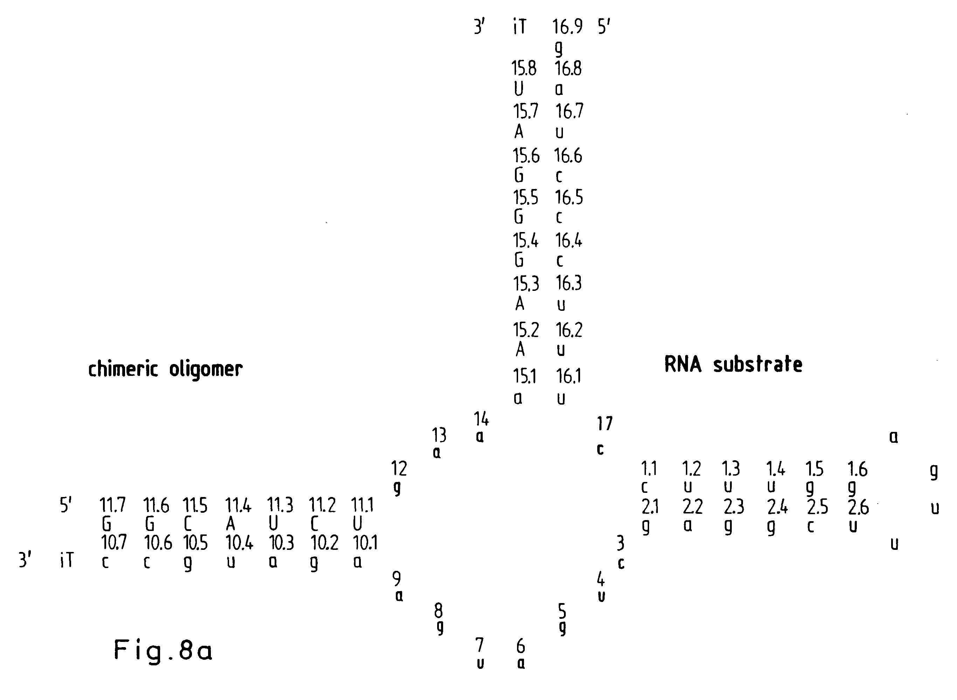

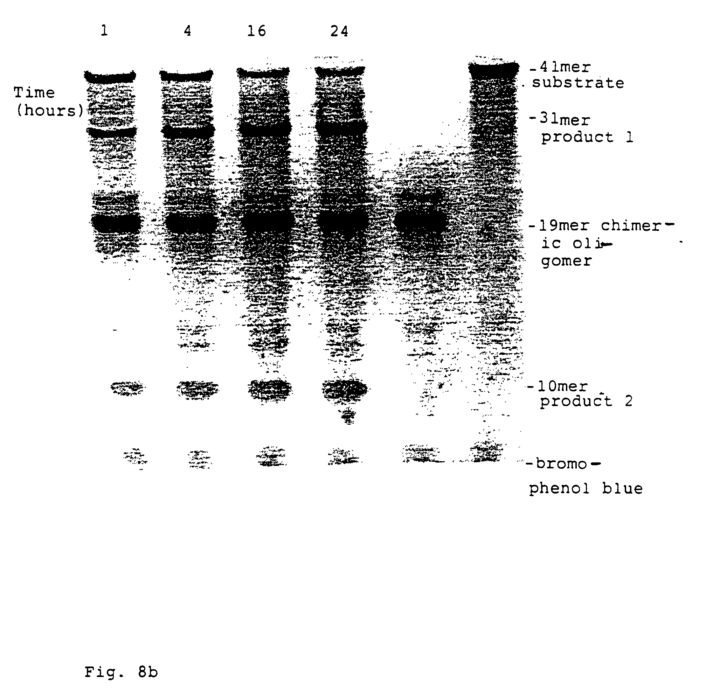

The reaction was carried out as described in example 1, except that the chimeric oligomer concentration was 10 µmol/l and the RNA substrate concentration was 2 µmol/l. The result is shown in Fig. 8b.

### Example 7

### Cleavage of an IL-1α RNA substrate

An IL-1α RNA substrate having the nucleotide sequence shown in SEQ ID NO. 11 and an RNA-cleaving oligomer having the nucleotide shown in SEQ ID NO. 12 were synthesized in an analogous manner to that described in example 1. The oligomer was synthesized with 2'-methoxy-modified nucleotides in the sequences outside the active centre, and with ribonucleotides in the active centre "GAAA".

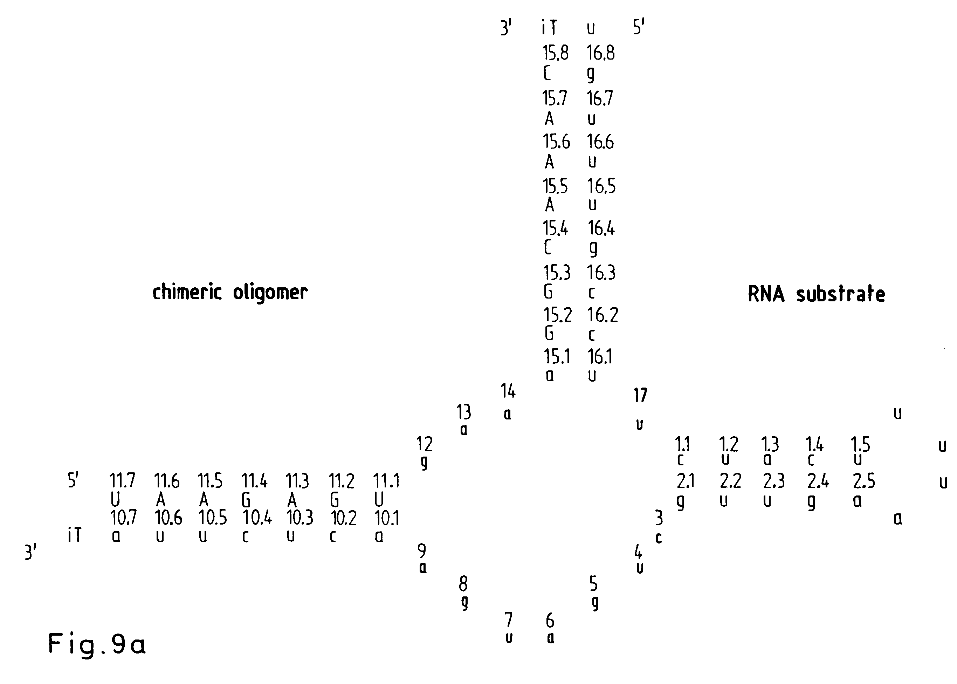

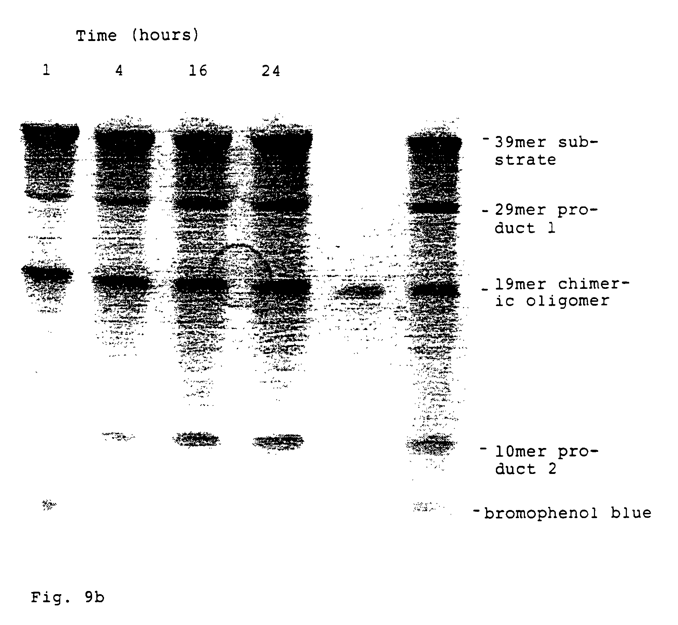

The reaction was carried out as described in example 1, except that the chimeric oligomer concentration was 10 µmol/l and the RNA substrate concentration was 2 µmol/l. The result is shown in Fig. 9b.

### Example 8

### Cleavage of a c-jun RNA substrate

A c-jun RNA substrate having the nucleotide sequence shown in SEQ ID NO. 13 and an RNA-cleaving oligomer having the nucleotide sequence shown in SEQ ID NO. 14 were synthesized in an analogous manner to that described in example 1. The oligomer was synthesized with 2'-methoxy-modified nucleotides in the sequences outside the active centre, and with ribonucleotides in the active centre "CUGAUGA".

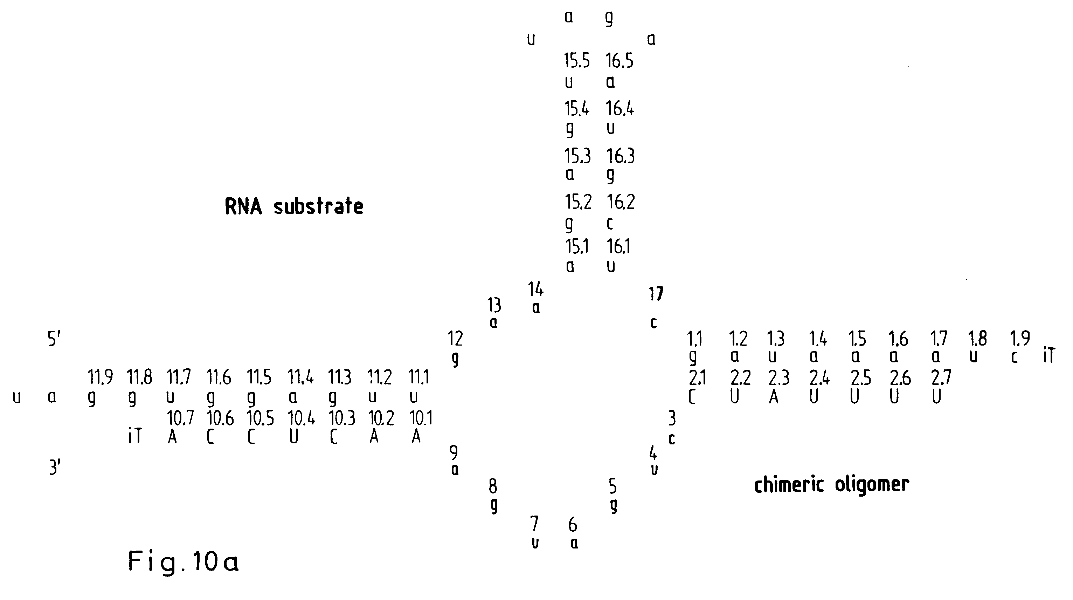

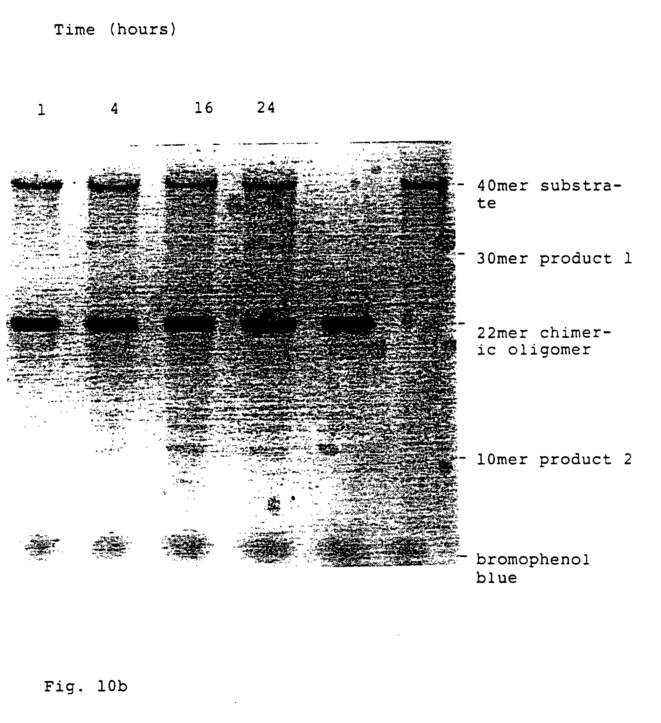

The reaction was carried out as described in example 1, except that the chimeric oligomer concentration was 10 µmol/l and the RNA substrate concentration was 2 µmol/l. The result is shown in Fig. 10b.

### Example 9

### Cleavage of an IL-1 receptor RNA substrate

An IL-1 receptor RNA substrate having the nucleotide sequence shown in SEQ ID NO. 15 and an RNA-cleaving oligomer having the nucleotide sequence shown in SEQ ID NO. 16 were synthesized in analogous manner to that described in example 1. The oligomer was synthesized with 2'-allyloxy-modified nucleotides in the sequences outside the active centre, and with ribonucleotides in the active centre "CUGAUGA".

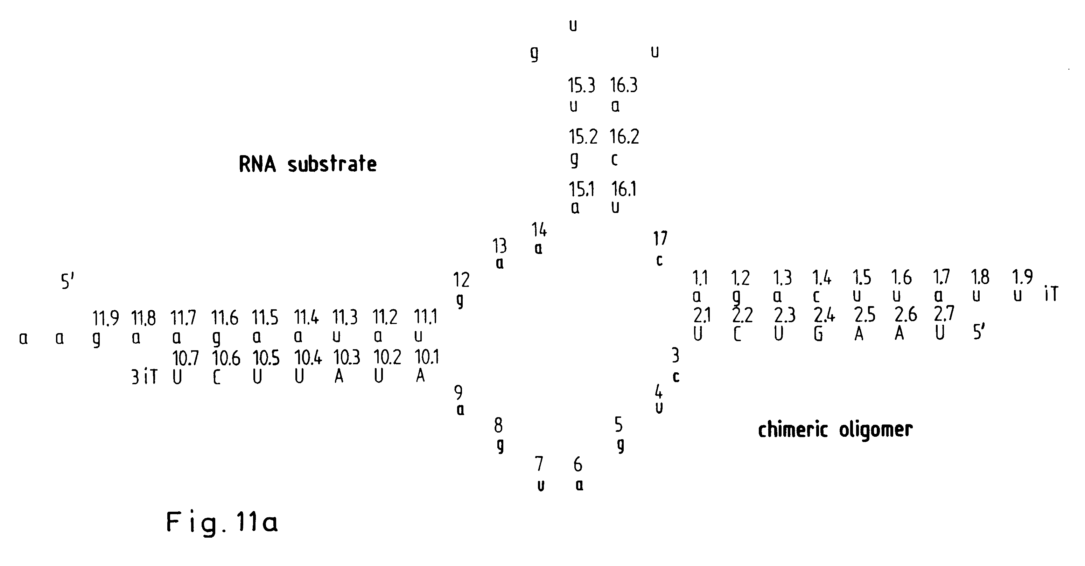

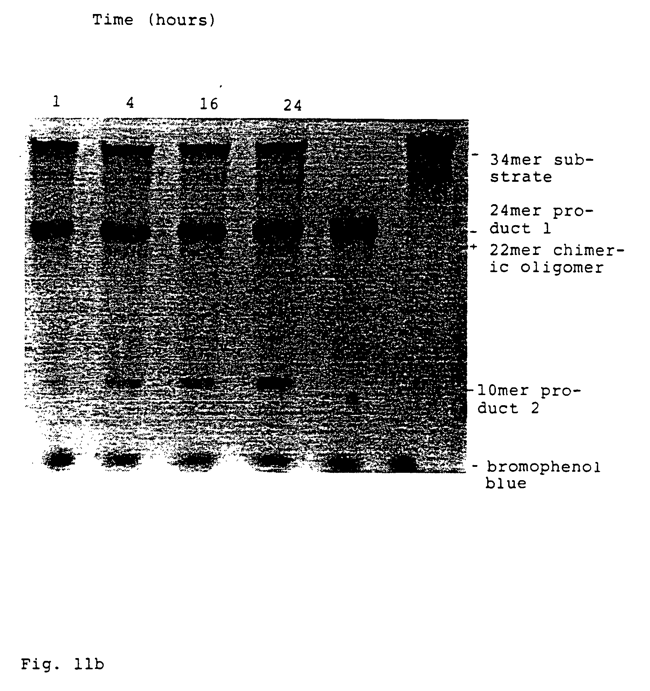

The reaction was carried out as described in example 1, except that the chimeric oligomer concentration was 10 µmol/l and the RNA substrate concentration was 2 µmol/l. The result is shown in Fig. 11b.

### Example 10

### Cleavage of an angiotensinogen RNA substrate

An angiotensinogen RNA substrate having the nucleotide sequence shown in SEQ ID N0. 17 and an RNA-cleaving oligomer having the nucleotide sequence shown in SEQ ID N0. 18 were synthesized in an analogous manner to that described in example 1. The oligomer was synthesized with 2'-methoxy-modified nucleotides in the sequences outside the active centre, and with ribonucleotides in the active centre "CUGAUGA".

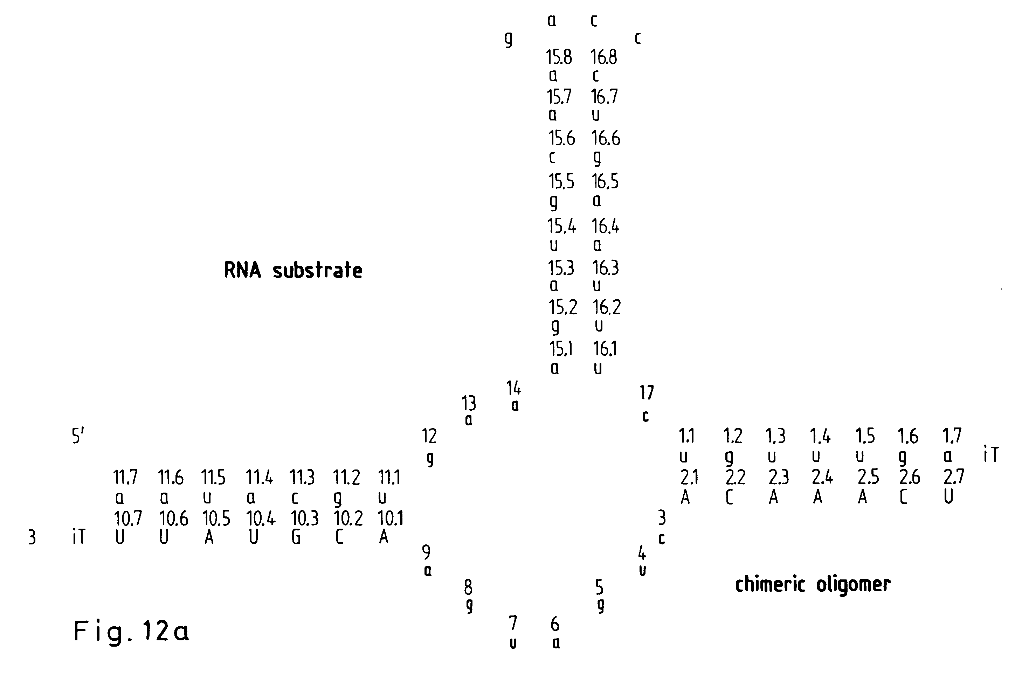

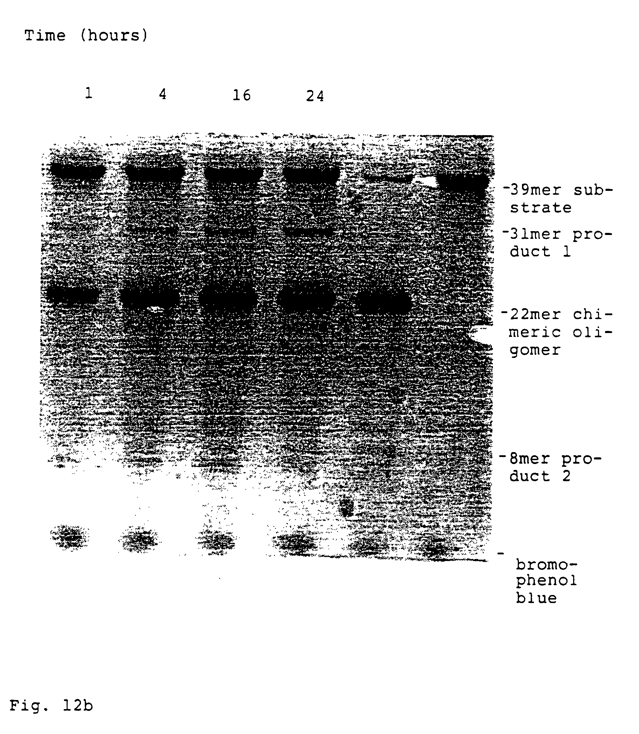

The reaction was carried out as described in example 1, except that the chimeric oligomer concentration was 10 µmol/l and the RNA substrate concentration was 2 µmol/l. The result is shown in Fig. 12b.

## Claims

1. Chimeric oligomer having an RNA-cleavage activity which has a structure according to formula (I) :
5'-X-Z-Y-3' (I)
in which X and Y represent oligomeric sequences containing building blocks composed of unmodified nucleotides or/and nucleotide analogues and contribute to the formation of a specific hybridisation of the oligomer with an RNA-substrate,
in which X and Y each contain at least one building block that differs from deoxyribonucleotides and ribonucleotides,
wherein Z represents the active centre of the chimeric oligomer having a structure of formulae (IIIa), (IIIb), (IVa) or (IVb) :
5'-G¹²A¹³A¹⁴R^{15.1}-3' (IIIa)
5'-N^{λ12}G¹²A¹³A¹⁴R^{15.1}-3' (IIIb)
5'-C³U⁴G⁵A⁶N⁷G⁸A⁹-3' (IVa)
5'-C³U⁴G⁵A⁶N⁷G⁸A⁹N^{9λ}-3' (IVb)
in which the building blocks of the structures (IIIa), (IIIb), (IVa), (IVb) are selected from unmodified nucleotides or/and nucleotide analogues,
in which the building blocks have a structure according to formula (II) : 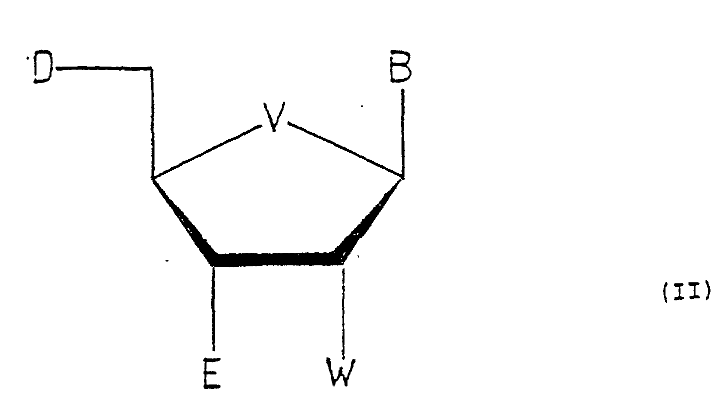
in which B denotes a residue, which is a naturally occurring nucleobase or a functional equivalent thereof, which is in particular selected from the group comprising adenin-9-yl (A), cytosin-1-yl (C), guanin-9-yl (G), uracil-1-yl (U), uracil-5-yl (ψ) hypoxanthin-9-yl (I)-, thymin-1-yl (T), 5-methyl- cytosin-1-yl (5MeC), 2,6-diaminopurin-9-yl (AminoA), purin-9-yl (P), 7-deazaadenin-9-yl (c⁷A), 7-deazaguanin-9-yl (c⁷G), 5-propynylcytosin-1-yl (5-pC), 5-propynyluracil-1-yl (5-pU), isoguanin-9-yl, 2-aminopurin-9-yl, 6-methyluracil-1-yl, 4-thiouracil-1-yl, 2-pyrimidone-1-yl and quinazoline-2,4-dione-1-yl;
V independently denotes an O, S, NH or CH₂-group,
W independently denotes an H or OH or a straight-chained or branched alkyl, alkoxy, alkenyl, alkenyloxy, alkynyl or alkynyloxy with 1 to 10 C-atoms, which can be optionally substituted by one or several halogen, cyano, amino, carboxy, ester, ether, carboxamide, hydroxyl or/and mercapto groups, or is selected from -COOH, -CONH₂, CONHR¹, CONR¹R², -NH₂, -NHR¹, -NR¹R², -NHCOR¹, -SH, SR¹, -F, -ONH₂, -ONHR¹, -ONR¹R², NHOH, -MHOR¹, -NR²OH and -NR²OR¹, in which R¹ and R² independently denote unsubstituted or substituted alkyl, alkenyl or alkynyl groups as defined above,
D and E denote residues which together form a phosphodiester or phosphorothioate diester bond between adjacent nucleosides or analogues or together form an analogue of an internucleosidic bond,
in which the structures (IIIa) or (IIIb) contain a total of one or two deoxyribonucleotides and the structures (IVa) or (IVb) contain a total of one, two, three, or four deoxyribonucleotides and
in which
G in each case independently denotes a building block according to formula (II), in which B is guanin-9-yl or a functional equivalent thereof for that particular location in the hammerhead core structure, which is preferably selected from I, c⁷G and isoguanin-9-yl;
A in each case independently denotes a building block according to formula (II), in which B is adenin-9-yl or a functional equivalent thereof for that particular location in the hammerhead core structure, which is preferably selected from aminoA, P, c⁷A and 2-aminopurin-9-yl;
U in each case independently denotes a building block according to formula (II), in which B is uracil-1-yl or a functional equivalent thereof for that particular location in the hammerhead core structure, which is preferably selected from ψ, T, 5-pU, 6-methyluracil-1-yl, 4-thiouracil-1-yl, 2-pyrimidone-1-yl and quinazoline-2,4-dione-1-yl;
C in each case independently denotes a building block according to formula (II), in which B is cytosin-1-yl or a functional equivalent thereof for that particular location in the hammerhead core structure, which is preferably selected from 5MeC, 5-pC and 2-pyrimidone-1-yl;
R in each case independently denotes a building block according to formula (II), in which B independently denotes G or A as defined above;
N in each case independently denotes a building block according to formula (II).

2. Oligomer as claimed in claim 1,
wherein
the regions X and Y do not contain any ribonucleotides at pyrimidine positions.

3. Oligomer as claimed in claim 1 or 2,
wherein
the regions X and Y do not contain any ribonucleotides.

4. Oligomer as claimed in one of the claims 1-3,
wherein
the regions X and Y are essentially composed of building blocks having the structure (II), in which W is in each case independently selected from optionally substituted alkyl, alkoxy, alkenyl, alkenyloxy, alkynyl or alkynyloxy residues with 1 to 10 C atoms.

5. Oligomer as claimed in one of the claims 1-4,
wherein
the regions X and Y each independently contain 3 to 40 building blocks.

6. Oligomer as claimed in one of the claims 1-5,
wherein
the active centre Z contains one or several building blocks having the structure (II), in which W in each case is independently selected from optionally substituted alkyl, alkoxy, alkenyl, alkenyloxy, alkynyl or alkynyloxy residues with 1 to 5 C atoms.

7. Oligomer as claimed in one of the claims 1-6,
wherein
the building blocks of the active centre Z are linked by phosphodiester bonds.

8. Oligomer as claimed in one of the claims 1-7,
wherein
the 3' end is protected against exonuclease degradation.

9. Oligomer as claimed in one of the claims 1-8,
wherein
the active centre Z has the structure (IIIa) or (IIIb) and has cleavage activity for an RNA-substrate that contains a structure of formulae (Va) or (Vb):
5'-U^{16.1}H¹⁷-S-C³U⁴G⁵A⁶N⁷G⁸A⁹R^{10.1}-3' (VA)
5'-U^{16.1}H¹⁷-S-C³U⁴G⁵A⁶N⁷G⁸A⁹N^{9λ}R^{10.1}-3' (Vb)
in which N, H, R, S, G, A, U and C represent ribonucleotides and
N denotes G, A, U or C;
H denotes A, C or U;
R denotes A or G and
S denotes an RNA-sequence capable of forming a hairpin structure.

10. Oligomer as claimed in claim 9,
wherein Z has the structure (IIIa) or (IIIb) and contains no more than two ribonucleotides; preferably no more than one ribonucleotide and more preferably no ribonucleotides and no deoxyribonucleotides.

11. Oligomer as claimed in one of the claims 9-10,
wherein G¹² denotes a ribonucleotide, a deoxyribonucleotide or a building block, in which W is a C₁-C₄-alkyl, alkenyl, alkoxy, or alkenyloxy group optionally substituted with OH, preferably a (2-hydroxyethoxy) residue.

12. Oligomer as claimed in one of the claims 9-11,
wherein A¹³, A¹⁴ or/and R^{15.1} are selected from building blocks, in which W is preferably a C₁-C₄-alkyl, alkenyl, alkoxy, or alkenyloxy group, optionally substituted with OH.

13. Oligomer as claimed in claim 12,
wherein W is selected from methoxy, (2-hydroxyethoxy) and allyloxy residues.

14. Oligomer as claimed in one of the claims 9-13,
wherein the RNA-substrate is selected from the group comprising human interleukin-2 mRNA, human ICAM-1 mRNA, human TGF-β mRNA, human tissue factor pre-mRNA, human protein kinase C-α mRNA, human factor KBF-1 mRNA, human 5-lipoxygenase mRNA, human interleukin-8 RNA, human interleukin-5 RNA, human interleukin-1 receptor mRNA, human interleukin-1-α mRNA, human 12-lipoxygenase mRNA, the transcript of the human cytomegalovirus DNA-polymerase gene and a transcript of the human papilloma virus type 8.

15. Oligomer as claimed in one of the claims 9-14,
wherein the RNA-substrate contains a region from human interleukin-2 mRNA having the nucleotide sequence shown in SEQ ID No. 1, human ICAM-1 mRNA having the nucleotide sequence shown in SEQ ID No. 3, human PKC-α RNA having the nucleotide sequence shown in SEQ ID No. 9, and human interleukin-1α mRNA having the nucleotide sequence shown in SEQ ID No. 11.

16. Oligomer as claimed in claim 15,
wherein the oligomer contains the nucleotide sequence shown in SEQ ID No. 2, SEQ ID No. 4, SEQ ID No. 10 or SEQ ID No. 12.

17. Oligomer as claimed in one of the claims 1-8,
wherein the catalytic centre Z has the structure (IVa) or (IVb) and exhibits cleavage activity for an RNA-substrate that contains a structure of formulae (VIa) or (VIb):
5'-Y^{11.1}G¹²A¹³A¹⁴R^{15.1}-S-U^{16.1}H¹⁷-3'1 (VIa)
5'-Y^{11.1}N^{λ12}G¹²A¹³A¹⁴R^{15.1}-S-U^{16.1}H¹⁷-3' (VIb)
in which N, H, Y, R, S, G, A, U and C represent ribonucleotides and
N denotes G, A, U or C,
H denotes A, C or U,
R denotes A or G,
Y denotes C or U and
S is an RNA sequence capable of forming a hairpin structure.

18. Oligomer as claimed in claim 17,
wherein Z has the structure (IVa) or (IVb) and contains no more than six ribonucleotides, preferably no pyrimidine ribonucleotides.

19. Oligomer as claimed in one of the claims 17-18,
wherein one or several building blocks at the positions G⁵, A⁶, N⁷, G⁸ and A⁹ are ribonucleotides, in which N⁷ is a purine base, in particular A.

20. Oligomer as claimed in one of the claims 17-19,
wherein C³, U⁴ and N^{λ12} are selected from building blocks, in which W is an optionally OH-substituted C₁-C₄ alkyl, alkenyl, alkoxy or alkenyloxy group or an NH₂ group.

21. Oligomer as claimed in claim 20,
wherein W is selected from methoxy, (2-hydroxyethyloxy), allyloxy, allyl and NH₂ residues.

22. Oligomer as claimed in one of the claims 17-21,
wherein the RNA-substrate is selected from the group comprising human interleukin-6 mRNA, human MDR-1 mRNA, human monocyte chemotactic protein RNA, human macrophage scavenger receptor type II mRNA, human macrophage scavenger receptor type I mRNA, human macrophageinflammatory protein-1α mRNA, human p53 RNA, human jun-B mRNA, human c-jun RNA, human interferon-γ type II mRNA, human hepatocyte growth factor mRNA, human HER2 mRNA, human Alzheimer's disease amyloid mRNA, human interleukin-1 mRNA, human interleukin-1 receptor mRNA, human 3-hydroxy-3-methylglutaryl coenzyme A reductase RNA, human angiotensinogen mRNA, human angiotensinconverting enzyme mRNA, human acyl-coenzyme A: cholesterol acyltransferase mRNA, human PDGF receptor mRNA, human TNF receptor mRNA, human TGF β mRNA, human NF-kappa B p65 subunit mRNA, human c-myc RNA, human 12-lipoxygenase mRNA, human interleukin-4 RNA, human interleukin-10 mRNA, human basic FGF mRNA, human EGF-receptor mRNA, human c-myb mRNA, human c-fos RNA, human bcl-2 mRNA, human bcl-1 mRNA, human ICAM-1 mRNA, a transcript of human papilloma virus type 11 and transcripts of human papilloma virus type 16 and type 18.

23. Oligomer as claimed in one of the claims 17-22,
wherein the RNA-substrate contains a region from human interleukin-6 mRNA having the nucleotide sequence shown in SEQ ID No. 5, human MDR-1 mRNA having the nucleotide sequence shown in SEQ ID No. 7, c-jun RNA having the nucleotide sequence shown in SEQ ID No. 13, human interleukin-1 receptor mRNA having the nucleotide sequence shown in SEQ ID No. 15, and human angiotensinogen RNA having the nucleotide sequence shown in SEQ ID No. 17.

24. Oligomer as claimed in claim 23,
wherein the oligomer contains the nucleotide sequence shown in SEQ ID No. 6, SEQ ID No. 8, SEQ ID No. 14, SEQ ID No. 16 and SEQ ID No. 18.

25. Oligomer as claimed in one of the claims 1-24,
wherein it is linked to a prosthetic group.

26. Pharmaceutical composition that contains one or several chimeric oligomers as claimed in one of the claims 1-25 as the active substance and optionally pharmaceutically acceptable auxiliary substances, additives and carriers.

27. Use of a chimeric oligomer as claimed in one of the claims 1-25 or a pharmaceutical composition as claimed in claim 26 to produce an agent for the specific inactivation of the expression of genes in eukaryotes, prokaryotes and viruses.

28. Use for preparing a medicament as claimed in claim 27 for the specific inactivation of the expression of human genes in a cell, for the specific inactivation of the expression of tumor genes, for the specific inactivation of the expression of viral genes or RNA molecules in a cell, or for the specific inactivation of the expression of plant genes.

29. Use as claimed in one of the claims 27-28,
wherein the active substance is administered at a concentration of 0.01 to 10,000 µg/kg body weight.

30. Use as claimed in one of the claims 27-29,
wherein the active substance is administered by injection, inhalation, as a spray, orally, topically or rectally.

31. Method for the specific in vitro inactivation of the expression of genes,
wherein a chimeric oligomer as claimed in one of the claims 1-25 is introduced into a cell in an active concentration so that the oligomer specifically cleaves a predetermined RNA molecule present in the cell.

32. Method as claimed in claim 31,
wherein the cleavage occurs catalytically.

33. Use of a chimeric oligomer as claimed in one of the claims 1-25 for the manufacture of a medicament for the specific inactivation of the expression of genes,
wherein said chimeric oligomer is introduced into a cell in an active concentration so that the oligomer specifically cleaves a predetermined RNA molecule present in the cell.

34. Use as claimed in claim 33,
wherein the cleavage occurs catalytically.

35. Use of a chimeric oligomer as claimed in one of the claims 1-25 as an RNA restriction enzyme, as a diagnostic agent, or to identify the function of unknown genes.

36. A kit for the restriction cleavage of RNA molecules,
wherein it contains a chimeric oligomer as claimed in one of the claims 1-25 and suitable buffer substances.

37. A method of preparing a composition for alleviating psoriasis, comprising formulating an effective amount of an oligomer selected from the group consisting of the oligomers of claims 1-13 and 17-21, wherein the RNA substrate is an IL-2 or ICAM-1 RNA.

38. A method of preparing a composition for alleviating common cold, comprising formulating an effective amount of an oligomer selected from the group consisting of the oligomers of claims 1-13 and 17-21, wherein the RNA substrate is an ICAM-1 RNA.

39. A method of preparing a composition for alleviating transplant rejection, comprising formulating an effective amount of an oligomer selected from the group consisting of the oligomers of claims 1-13 and 17-21,
wherein the RNA substrate is an ICAM-1 RNA.

40. A method of preparing a composition for alleviating Kaposi's sarcoma, comprising formulating an effective amount of an oligomer selected from the group consisting of the oligomers of claims 1-13 and 17-21, wherein the RNA substrate is an IL-6 RNA.

41. A method of preparing a composition for treating cancer, comprising formulating an effective amount of an oligomer selected from the group consisting of the oligomers of claims 1-13 and 17-21, wherein the RNA substrate is a PKC-α RNA.

42. A method of preparing a composition for treating hypertension, comprising formulating an effective amount of an oligomer selected from the group consisting of the oligomers of claims 1-13 and 17-21, wherein the RNA substrate is an angiotensinogen RNA.

## Patentansprüche

1. Chimäres Oligomer mit einer Struktur gemäß Formel (I), das RNS-spaltende Aktivität besitzt:
5'-X-Z-Y-3' (I°
wobei X und Y oligomere Sequenzen darstellen, die Bausteine enthalten, die aus nicht modifizierten Nukleotiden oder/und Nukleotidanalogen zusammengesetzt sind und die zur Bildung einer spezifischen Hybridisierung des Oligomeren mit einem RNS-Substrat beitragen,
wobei X und Y jeweils mindestens einen Baustein enthalten, der sich von Desoxyribonukleotiden und Ribonukleotiden unterscheidet,
wobei Z das aktive Zentrum des chimären Oligomers mit einer Struktur gemäß den Formeln (IIIa), (IIIb), (IVa) oder (IVb) darstellt:
5'-G¹²A¹³A¹⁴R^{15.1}-3' (IIIa)
5'-N^{λ12}G¹²A¹³A¹⁴R^{15.1}-3' (IIIb)
5'-C³U⁴G⁵A⁶N⁷G⁸A⁹-3' (IVa)
5'-C³U⁴G⁵A⁶N⁷G⁸A⁹N^{9λ}-3' (IVb)
wobei die Bausteine mit den Strukturen (IIIa), (IIIb), (IVa), (IVb) aus nicht modifizierten Nukleotiden oder/und Nukleotidanalogen ausgewählt sind,
wobei die Bausteine eine Struktur gemäß der Formel (II) haben: 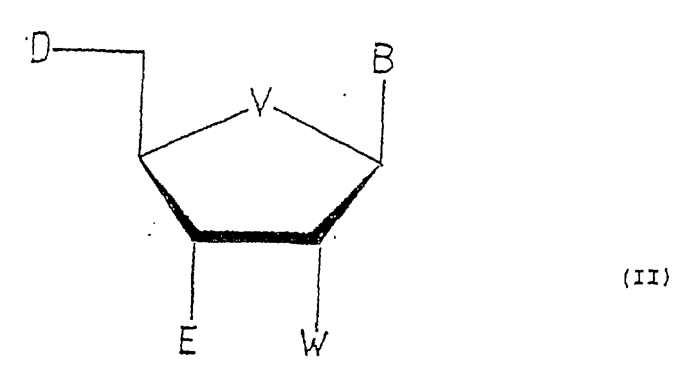
worin B für einen Rest steht, der eine natürlich vorkommende Nukleobase oder ein funktionales Äquivalent davon ist, der insbesondere aus der Gruppe ausgewählt wird, welche Adenin-9-yl (A), Cytosin-1-yl (C), Guanin-9-yl (G), Uracil-1-yl (U), Uracil-5-yl (ψ), Hypoxanthin-9-yl (I), Thymin-1-yl (T), 5-Methyl-cytosin-1-yl (5MeC), 2,6-Diaminopurin-9-yl (AminoA), Purin-9-yl (P), 7-Deazaadenin-9-yl (c⁷A), 7-Deazaguanin-9-yl (c⁷G), 5-Propynylcytosin-1-yl (5-pC), 5-Propynyluracil-1-yl (5-pU), Isoguanin-9-yl, 2-Aminopurin-9-yl, 6-Methyluracil-1-yl, 4-Thiouracil-1-yl, 2-Pyrimidon-1-yl und Quinazolin-2,4-dion-1-yl enthält;
V bedeutet unabhängig eine 0-, S-, NH- oder CH₂-Gruppe,
W bedeutet unabhängig ein H oder OH oder ein geradkettiges oder verzweigtes Alkyl, Alkoxy, Alkenyl, Alkenyloxy, Alkynyl oder Alkynyloxy mit 1 bis 10 C-Atomen, welches gegebenenfalls mit einer oder mehreren Halogen, Cyano, Amino, Carboxy,
Ester, Ether, Carboxamid, Hydroxyl oder/und Mercapto-Gruppen substituiert sein kann, oder wird aus -COOH, -CONH₂, CONHR¹, CONR¹R², -NH₂, -NHR¹, -NR¹R², -NHCOR¹, -SH, SR¹, -F, -ONH₂,-ONHR¹ , -ONR¹R², NHOH, -NHOR¹, NR²OH und - NR²OR¹ ausgewählt,
wobei R¹ und R² unabhängig voneinander unsubstituierte oder substituierte Alkyl-, Alkenyl- oder Alkynyl-Gruppen wie oben definiert bedeuten,
D und E sind Reste, welche zusammengenommen eine Phosphodiester- oder Phosphorothioatdiesterbindung zwischen benachbarten Nucleosiden oder deren Analogen bilden, oder welche zusammengenommen ein Analog einer internukleosidischen Bindung bilden, wobei die Strukturen (IIIa) oder (IIIb) insgesamt ein oder zwei Desoxyribonukleotide enthalten und die Strukturen (IVa) oder (IVb) insgesamt ein, zwei, drei oder vier Desoxyribonukleotide enthalten, und worin
G jeweils unabhängig einen Baustein gemäß der Formel (II) bedeutet, worin B ein Guanin-9-yl oder ein funktionales Äquivalent davon für jene bestimmte Position in der Kernstruktur des Hammerkopfs ist, welches bevorzugt aus I, c⁷G und Isoguanin-9-yl ausgewählt ist;
A jeweils unabhängig eine Baustein gemäß der Formel (II) bedeutet, worin B ein Adenin-9-yl oder ein fuktionales Äquivalent davon für jene bestimmte Position in der Kernstruktur des Hammerkopfs ist, welches bevorzugt aus AminoA, P, c⁷A und 2-Aminopurin-9-yl ausgewählt ist;
U jeweils unabhängig ein Baustein gemäß der Formel (II) bedeutet, worin B ein Uracil-1-yl oder ein fuktionales Äquivalent davon für jene bestimmte Position in der Kernstruktur des Hammerkopfs ist, welches bevorzugt aus Ψ, T, 5-pU, 6-Methyluracil-1-yl, 4-Thiouracil-1-yl, 2-Pyrimidone-1-yl und Quinazoline-2,4-dion-1-yl ausgewählt ist;
C jeweils unabhängig ein Baustein gemäß der Formel (II) bedeutet, worin B ein Cytosin-1-yl oder ein fuktionales Äquivalent davon für jene bestimmte Position in der Kernstruktur des Hammerkopfs ist, welches bevorzugt aus 5MeC, 5-pC und 2-pyrimidone-1-yl ausgewählt ist;
R jeweils unabhängig einen Baustein gemäß der Formel (II) bedeutet, worin B unabhängig als G oder A wie oben definiert ist;
N jeweils unabhängig einen Baustein gemäß der Formel (II) bedeutet.

2. Oligomer gemäß Anspruch 1, wobei die Bereiche X und Y keine Ribonukleotide an Positionen mit Pyrimidin enthalten.

3. Oligomer gemäß Anspruch 1 oder 2, wobei die Bereiche X und Y keine Ribonukleotide enthalten.

4. Oligomer gemäß einem der Ansprüche 1-3, wobei die Bereiche X und Y hauptsächlich aus Bausteinen mit der Struktur (II) bestehen, wobei W jeweils unabhängig aus gegebenenfalls substituiertem Alkyl-, Alkoxy-, Alkenyl-, Alkenyloxy-, Alkynyl- oder Alkynyloxyresten mit 1 bis 10 C Atomen ausgewählt ist.

5. Oligomer gemäß einem der Ansprüche 1-4, wobei die Bereiche X und Y jeweils unabhängig 3 bis 40 Bausteine enthalten.

6. Oligomer gemäß einem der Ansprüche 1-5, wobei das aktive Zentrum Z einen oder mehrere Bausteine mit der Struktur (II) enthält, worin W jeweils unabhängig aus gegebenenfalls substituierten Alkyl-, Alkoxy-, Alkenyl-, Alkenyloxy-, Alkynyl- oder Alkynyloxyresten mit 1 bis 5 C Atomen ausgewählt ist.

7. Oligomer gemäß einem der Ansprüche 1-6, wobei die Bausteine des aktiven Zentrums Z durch Phosphodiesterbindungen verknüpft sind.

8. Oligomer gemäß einem der Ansprüche 1-7, wobei das 3' Ende gegen den Abbau durch Exonuklease geschützt ist.

9. Oligomer gemäß einem der Ansprüche 1-8, wobei das aktive Zentrum Z die Struktur (IIIa) oder (IIIb) hat und Spaltungsaktivität gegenüber einem RNS-Substrat aufweist, das eine Struktur gemäß den Formeln (Va) oder (Vb) enthält:
5'-U^{16.1}H¹⁷-S-C³U⁴G⁵A⁶N⁷G⁸A⁹R^{10.1}-3' (VA)
5'-U^{16.1}H¹⁷-S-C³U⁴G⁵A⁶N⁷G⁸A⁹N^{9λ}R^{10.1}-3' (Vb)
worin N, H, R, S, G, A, U und C Ribonukleotide darstellen und
N G,A,U oder C bedeutet;
H A, C oder U bedeutet;
R A oder G bedeutet und
S eine RNS-Sequenz bedeutet, die fähig ist, eine Haarnadel Struktur zu bilden.

10. Oligomer gemäß Anspruch 9, wobei Z die Struktur (IIIa) oder (IIIb) hat und nicht mehr als zwei Ribonukleotide enthält, bevorzugt nicht mehr als ein Ribonukleotid und noch bevorzugter keine Ribonukleotide und keine Desoxyribonukleotide enthält.

11. Oligomer gemäß einem der Ansprüche 9-10,
wobei G¹² ein Ribonukleotid, ein Desoxyribonukleotid oder einen Baustein bedeutet, wobei W eine C₁-C₄ Alkyl-, Alkenyl-, Alkoxy-, oder Alkenyloxygruppe ist, die gegebenenfalls mit OH-, bevorzugt mit einem (2-Hydroxyethoxy) Rest substituiert ist.

12. Oligomer gemäß einem der Ansprüche 9-11,
wobei A¹³, A¹⁴ oder/und R^{15.1} aus Bausteinen ausgewählt sind, in denen W bevorzugt eine gegebenenfalls mit OH substituierte C₁-C₄ -Alkyl-, Alkenyl-, Alkoxy-, oder Alkenyloxygruppe ist.

13. Oligomer gemäß Anspruch 12,
wobei W aus Methoxy-, (2-Hydroxyethoxy)- und Allyloxyresten ausgewählt ist.

14. Oligomer gemäß einem der Ansprüche 9-13,
wobei das RNS-Substrat aus einer Gruppe, die menschliche Interleukin-2 mRNS, menschliche ICAM-I mRNS, menschliche TGF-β mRNS, menschliche Gewebefaktor pre-mRNS, menschliche Proteinkinasen C-α mRNS, menschliche Faktor KBF-1 mRNS, menschliche 5-Lipoxygenasen mRNS, menschliche Interleukin-8 RNS, menschliche Interleukin-5 RNS, menschliche Interleukin-1 Rezeptor mRNS, menschliche Interleukin-1-α mRNS, menschliche 12-Lipoxygenase mRNS, das Transkript des DNA-Polymerase Gens des menschlichen Zytomegalovirus und ein Transkript des menschlichen Papilloma Virus Typ 8 umfasst, ausgewählt wird.

15. Oligomer gemäß einem der Ansprüche 9-14,
wobei das RNS-Substrat einen Bereich der menschlichen Interleukin-2 mRNS, die die in SEQ ID No. 1 gezeigte Nukleotidsequenz hat, der menschlichen ICAM-I mRNS, die die in SEQ ID No. 3 gezeigte Nukleotidsequenz hat, der menschlichen PKC-α RNS, die die in SEQ ID No. 9 gezeigte Nukleotidsequenz hat, und der menschlichen Interleukin-1α mRNS, die die in SEQ ID No. 11 gezeigte Nukleotidsequenz hat, enthält.

16. Oligomer gemäß Anspruch 15,
worin das Oligomer die in SEQ ID No. 2, SEQ ID No. 4, SEQ ID No. 10 oder SEQ ID No. 12 gezeigte Nucleotidsequenz hat.

17. Oligomer gemäß einem der Ansprüche 1-8,
wobei das katalytische Zentrum Z die Struktur (IVa) oder (IVb) hat und eine Spaltungsaktivität gegenüber einem RNS-Substrat zeigt, das eine Struktur der Formeln (VIa) oder (VIb) enthält:
5'-Y^{11.1}G¹²A¹³A¹⁴R^{15.1}-S-U^{16.1}H¹⁷-3'1 (VIa)
5'-Y^{11.1}N^{λ12}G¹²A¹³A¹⁴R^{15.1}-S-U^{16.1}H¹⁷-3' (VIb)
wobei N, H, Y, R, S, G, A, U und C Ribonukleotide darstellen und
N G, A, U oder C bedeutet,
H A, C oder U bedeutet,
R A oder G bedeutet,
Y C oder U bedeutet und
S eine RNS Sequenz ist, die fähig ist, eine Haarnadelstruktur zu bilden.

18. Oligomer gemäß Anspruch 17,
wobei Z die Struktur (IVa) oder (IVb) hat und nicht mehr als sechs Ribonukleotide, bevorzugt keine
Pyrimidinribonukleotide, enthält.

19. Oligomer gemäß einem der Ansprüche 17-18,
wobei ein oder mehrere Bausteine an den Positionen G⁵, A⁶,
N⁷, G⁸ und A⁹ Ribonukleotide sind, in denen N⁷ eine Purinbase, insbesondere A ist.

20. Oligomer gemäß einem der Ansprüche 17-19,
wobei C³, U⁴ und N^{λ12} aus Bausteinen ausgewählt sind, worin W eine gegebenenfalls mit OH-substituierte C₁-C₄-Alkyl-,
Alkenyl-, Alkoxy- oder Alkenyloxygruppe, oder eine NH₂ Gruppe ist.

21. Oligomer gemäß Anspruch 20,
wobei W aus Methoxy-, (2-Hydroxyethyloxy)-, Allyloxy-, Allyl- und NH₂ Resten ausgewählt ist.

22. Oligomer gemäß einem der Ansprüche 17-21,
wobei das RNS-Substrat aus der Gruppe, die menschliche Interleukin-6 mRNS, menschliche MDR-I mRNS, menschliche chemotaktisches Monozyten Protein RNS, menschliche Macrophagen Scavenger Rezeptor Typ II mRNS, menschliche Macrophagen Scavenger Rezeptor Typ I mRNS, menschliche Macrophagen Entzündungsprotein-1α mRNS, menschliche p53 RNS, menschliche jun-B mRNS, menschliche c-jun RNS, menschliche Interferon-γ Typ II mRNS, menschliche Hepatozyten Wachstumsfaktor mRNS, menschliche HER2 mRNS, menschliche Alzheimer's Krankheit Amyloid mRNS, menschliche Interleukin-1 mRNS, menschliche Interleukin-1 Rezeptor mRNS, menschliche 3-Hydroxy-3-methylglutaryl Coenzym A Reduktase RNS, menschliche Angiotensinogen mRNS, menschliche angiotensinumwandelndes Enzym mRNS, menschliches Acylcoenzyme A: Cholesterin Acyltransferasen mRNS, menschliche PDGF Rezeptoren mRNS, menschliche TNF Rezeptoren mRNS, menschliche TGF β mRNS, menschliche NF-kappa B p65 Untereinheit mRNS, menschliche c-myc RNS, menschliche 12-Lipoxygenasen mRNS, menschliche Interleukin-4 RNS, menschliche Interleukin-10 mRNS, menschliche basic FGF mRNS, menschliche EGF-Rezeptoren mRNS, menschliche c-myb mRNS, menschliche c-fos RNS, menschliche bcl-2 mRNS, menschliche bcl-1 mRNS, menschliche ICAM-1 mRNS, ein Transkript des menschlichen Papillomavirus Typ 11 und Transkripte des menschlichen Papillomavirus Typ 16 und Typ 18 umfasst, ausgewählt wird.

23. Oligomer gemäß einem der Ansprüche 17-22,
wobei das RNS-Substrat einen Bereich der menschlichen Interleukin-6 mRNS enthält, die die in der SEQ ID No. 5 gezeigte Nukeotidsequenz hat, menschliche MDR-1 mRNS, die die in der SEQ ID No. 7 gezeigte Nukeotidsequenz besitzt, c-jun RNS, die die in der SEQ ID No. 13 gezeigte Nukeotidsequenz hat, menschliche Interleukin-1 Rezeptoren mRNS, die die in der SEQ ID No. 15 gezeigte Nukeotidsequenz hat, und menchliche Angiotensinogen RNS, die die in der SEQ ID No. 17 gezeigte Nukeotidsequenz hat.

24. Oligomer gemäß Anspruch 23,
wobei das Oligomer die in der SEQ ID No. 6, SEQ ID No. 8, SEQ ID No. 14, SEQ ID No. 16 und SEQ ID No. 18 gezeigte Nukeotidsequenz enthält.

25. Oligomer gemäß einem der Ansprüche 1-24,
wobei es mit einer prosthetischen Gruppe verknüpft ist.

26. Pharmazeutische Zusammensetzung, die ein oder mehrere chimäre Oligomere gemäß einem der Ansprüche 1-25 als aktive Substanz enthält und gegebenenfalls pharmazeutisch akzeptable Hilfsstoffe, Zusatzstoffe und Träger enthält.

27. Verwendung eines chimären Oligomers gemäß einem der Ansprüche 1-25 oder einer pharmazeutischen Zusammensetzung gemäß Anspruch 26 zur Herstellung eines Agenzes zur spezifischen Deaktivierung der Exprimierung von Genen in Eukaryonten, Prokaryonten und Viren.

28. Verwendung zur Herstellung eines Medikaments gemäß Anspruch 27 zur spezifischen Deaktivierung der Exprimierung von menschlichen Genen in einer Zelle, zur spezifischen Deaktivierung der Exprimierung von Tumorgenen, zur spezifischen Deaktivierung der Exprimierung von viralen Genen oder von RNS-Molekülen in einer Zelle, oder zur spezifischen Deaktivierung der Exprimierung von Pflanzengenen.

29. Verwendung gemäß einem der Ansprüche 27-28,
wobei die aktive Substanz in einer Konzentration von 0,01 bis 10.000 µg/kg Körpergewicht verabreicht wird.

30. Verwendung gemäß einem der Ansprüche 27-29,
wobei die aktive Substanz durch Injektion, Inhalierung, als Spray, oral, topisch oder rektal verabreicht wird.

31. Verfahren zur spezifischen in vitro Deaktivierung der Exprimierung von Genen, wobei ein chimäres Oligomer gemäß einem der Ansprüche 1-25 in einer aktiven Konzentration in eine Zelle eingeführt wird, so daß das Oligomer ein vorgegebenes, sich in der Zelle befindendes RNS Molekül spezifisch spaltet.

32. Verfahren gemäß Anspruch 31, wobei die Spaltung katalytisch erfolgt.

33. Verwendung eines chimären Oligomers gemäß einem der Ansprüche 1-25 zur Herstellung eines Medikaments zur spezifischen Deaktivierung der Exprimierung von Genen, wobei das chimäre Oligomer in einer aktiven Konzentration in eine Zelle eingeführt wird, so daß das Oligomer ein vorgegebenes, sich in der Zelle befindendes RNS Molekül spezifisch spaltet.

34. Verwendung gemäß Anspruch 33, wobei die Spaltung katalytisch erfolgt.

35. Verwendung eines chimären Oligomers gemäß einem der Ansprüche 1-25 als RNS Restriktionsenzym, als diagnostisches Agenz, oder zur Identifizierung der Funktion unbekannter Gene.

36. Kit zur Restriktionsspaltung von RNS Molekülen, wobei es ein chimäres Oligomer gemäß einem der Ansprüche 1-25 und geeignete Buffersubstanzen enthält.

37. Verfahren zur Herstellung einer Zusammensetzung zur Milderung von Psoriasis, das umfasst das Formulieren einer wirksamen Menge eines aus der Gruppe, die aus den Oligomeren gemäß den Ansprüchen 1-13 und 17-21 ausgewählten Oligomeren besteht, wobei das RNS-Substrat eine IL-2- oder ICAM-1 RNS ist.

38. Verfahren zur Herstellung einer Zusammensetzung zur Milderung der gemeinen Erkältung, das umfasst das Formulieren einer wirksamen Menge eines aus der Gruppe bestehend aus den Oligomeren gemäß den Ansprüchen 1-13 und 17-21 ausgewählten Oligomers, wobei das RNS-Substrat eine ICAM-1 RNS ist.

39. Verfahren zur Herstellung einer Zusammensetzung zur Milderung von Transplantatabstoßung, das umfasst das Formulieren einer wirksamen Menge eines aus der Gruppe bestehend aus den Oligomeren gemäß den Ansprüchen 1-13 und 17-21 ausgewählten Oligomeren, wobei das *RNS-*Substrat eine ICAM-1 RNS ist.

40. Verfahren zur Herstellung einer Zusammensetzung zur Milderung von Kaposi's Sarcoma, das umfasst das Formulieren einer wirksamen Menge eines aus der Gruppe bestehend aus den Oligomeren gemäß den Ansprüchen 1-13 und 17-21 ausgewählten Oligomeren, wobei das RNS-Substrat eine IL-6 RNS ist.

41. Verfahren zur Herstellung einer Zusammensetzung zur Behandlung von Krebs, das umfasst das Formulieren einer wirksamen Menge eines aus der Gruppe bestehend aus den Oligomeren gemäß den Ansprüchen 1-13 und 17-21 ausgewählten Oligomeren, wobei das RNS-Substrat eine PKC-α RNS ist.

42. Verfahren zur Herstellung einer Zusammensetzung zur Behandlung von Bluthochdruck, das umfasst das Formulieren einer wirksamen Menge eines aus der Gruppe bestehend aus den Oligomeren gemäß den Ansprüchen 1-13 und 17-21 ausgewählten Oligomeren, wobei das RNS-Substrat eine Angiotensin RNS ist.

## Revendications

1. Oligomère chimérique ayant une activité de coupure d'ARN et possédant une structure selon la formule (I) :
5'-X-Z-Y-3' (I)
dans laquelle X et Y représentent des séquences contenant des éléments structuraux composés de nucléotides non modifiés et/ou d'analogues de nucléotides et contribuent à la formation d'une hybridation spécifique de l'oligomère avec un substrat d'ARN,
dans laquelle X et Y contiennent chacun au moins un élément structural qui diffère des désoxyribonucléotides et des ribonucléotides,
dans laquelle Z représente le centre actif de l'oligomère ayant une structure selon les formules (IIIa), (IIIb), (IVa) ou (IVb) :
5'-G¹²A¹³A¹⁴R^{15.1}-3' (IIIa)
5'-N^{λ12}G¹²A¹³A¹⁴R^{15.1}-3' (IIIb)
5'-C³U⁴G⁵A⁶N⁷G⁸A⁹-3' (IVa)
5'-C³U⁴G⁵A⁶N⁷G⁸A⁹N⁹-3' (IVb)
dans lesquelles les éléments structuraux des structures (IIIa), (IIIb), (IVa), (IVb) sont choisis parmi des nucléotides non modifiés et/ou des analogues de nucléotides,
dans laquelle les éléments structuraux ont une structure selon la formule (II) : 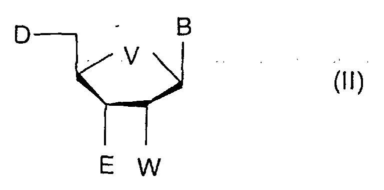
dans laquelle :
B indique un résidu qui est une nucléobase présente à l'état naturel ou un équivalent fonctionnel de celle-ci, qui est en particulier choisi dans le groupe comprenant les adénin-9-yle (A), cytosin-1-yle (C), guanin-9-yle (G), uracil-1-yle (U), uracil-5-yle (ψ), hypoxanthin-9-yle (I), thymin-1-yle (T), 5-méthylcytosin-1-yle (5MeC), 2,6-diaminopurin-9-yle (AminoA), purin-9-yle (P), 7-déaza-adénin-9-yle (c7A), 7-déazaguanin-9-yle (c⁷G), 5-propynylcytosin-1-yle (5-pC), 5-propynyluracil-1-yle (5-pU), isoguanin-9-yle, 2-aminopurin-9-yle, 6-méthyluracyl-1-yle, 4-thiouracil-1-yle, 2-pyrimidone-1-yle et quinazoline-2,4-dione-1-yle ;
V indique indépendamment un groupe O, S, NH ou CH₂,
W indique indépendamment un groupe H ou OH ou un alkyle, alcoxy, alcényle, alcényloxy, alcynyle ou alcynyloxy à chaîne linéaire ou ramifiée ayant de 1 à 10 atomes de carbone, qui peut être le cas échéant substitué par un ou plusieurs groupes halogène, cyano, amino, carboxy, ester, éther, carboxamide, hydroxyle et/ou mercapto, ou bien est choisi parmi -COOH, -CONH₂, -CONHR¹, -CONR¹R², -NH₂,-NR¹, -NR¹R², -NHCOR¹ -SH, -SR¹, -F, -ONH₂, -ONHR^{1,} -ONR¹R², -NHOH,-NHOR¹, -NR²OH et -NR²OR¹, où R¹ et R² indiquent indépendamment des groupes alkyle, alcényle ou alcynyle non substitués ou substitués tels que définis ci-dessus,
D et E indiquent des résidus qui forment ensemble un phosphodiester ou une liaison diester phosphorothioate entre des nucléosides ou analogues adjacents ou bien qui forment ensemble un analogue d'une liaison internucléosidique,
dans laquelle les structures (IIIa) ou (IIIb) contiennent au total un ou deux désoxyribonucléotides et les structures (IVa) ou (IVb) contiennent au total un, deux, trois, ou quatre désoxyribonucléotides, et
dans laquelle G indique indépendamment dans chaque cas un élément structural selon la formule (II), dans laquelle B est le guanin-9-yle ou un équivalent fonctionnel de celui-ci pour cet emplacement particulier dans la structure de noyau en tête de marteau, qui est de préférence choisi parmi I, c⁷G et isoguanin-9-yle ;
A indique indépendamment dans chaque cas un élément structural selon la formule (II), dans laquelle B est l'adénin-9-yle ou un équivalent fonctionnel de celui-ci pour cet emplacement particulier de la structure de noyau en tête de marteau, qui est de préférence choisi parmi aminoA, P, c⁷A et 2-aminopurin-9-yle ;
U indique indépendamment dans chaque cas un élément structural selon la formule (II), dans laquelle B est l'uracil-1-yle ou un équivalent fonctionnel de celui-ci pour cet emplacement particulier de la structure de noyau en tête de marteau, qui est de préférence choisi parmi ψ, T, 5-pU, 6-méthyluracil-1-yle, 4-thiouracil-1-yle, 2-pyrimidone-1-yle et quinazoline-2,4-dione-1-yle ;
C indique indépendamment dans chaque cas un élément structural selon la formule (II), dans laquelle B est le cytosin-1-yle ou un équivalent fonctionnel de celui-ci pour cet emplacement particulier de la structure de noyau en tête de marteau, qui est de préférence choisi parmi 5MeC, 5-pC et 2-pyrimidone-1-yle ;
R indique indépendamment dans chaque cas un élément structural selon la formule (II), dans laquelle B indique indépendamment G ou A tel que défini ci-dessus ;
N indique indépendamment dans chaque cas un élément structural selon la formule (II).

2. Oligomère selon la revendication 1, dans lequel les régions X et Y ne contiennent aucun ribonucléotide dans les positions des pyrimidines.

3. Oligomère selon la revendication 1 ou la revendication 2, dans lequel les régions X et Y ne contiennent aucun ribonucléotide.

4. Oligomère selon l'une quelconque des revendications 1 à 3, dans lequel les régions X et Y sont essentiellement composées d'éléments structuraux ayant la structure (II), dans laquelle W est choisi indépendamment dans chaque cas parmi des résidus alkyle, alcoxy, alcényle, alcényloxy, alcynyle ou alcynyloxy, le cas échéant substitués, ayant de 1 à 10 atomes de carbone.

5. Oligomère selon l'une quelconque des revendication 1 à 4, dans lequel les régions X et Y contiennent chacune indépendamment de 3 à 40 éléments structuraux.

6. Oligomère selon l'une quelconque des revendications 1 à 5, dans lequel le centre actif Z contient un ou plusieurs éléments structuraux ayant la structure (II), dans laquelle W est choisi indépendamment dans chaque cas parmi des résidus alkyle, alcoxy, alcényle, alcényloxy, alcynyle ou alcynyloxy, éventuellement substitués, ayant de 1 à 5 atomes de carbone.

7. Oligomère selon l'une quelconque des revendications 1 à 6, dans lequel les éléments structuraux du centre actif Z sont liés par des liaisons phosphodiester.

8. Oligomère selon l'une quelconque des revendications 1 à 7, dans lequel l'extrémité 3' est protégée contre la dégradation par une exonucléase.

9. Oligomère selon l'une quelconque des revendications 1 à 8, dans lequel le centre actif Z a la structure (IIIa) ou (IIIb) et possède une activité de coupure pour un substrat d'ARN qui contient une structure de formule (Va) ou (Vb) :
5'-U^{16.1}H¹⁷S-C³U⁴G⁵A⁶N⁷G⁸A⁹R^{10.1}-3' (Va)
5'-U^{16.1}H¹⁷-S-C³U⁴G⁵A⁶N⁷G⁸A⁹N^{9λ}R^{10.1}-3' (Vb)
dans laquelle :
N, H, R, S, G, A, U et C représentent des ribonucléotides, et
N indique G, A, U ou C ;
H indique A, C ou U ;
R indique A ou G ;
S indique une séquence d'ARN capable de former une structure en épingle à cheveux.

10. Oligomère selon la revendication 9, dans lequel la structure (IIIa) ou (IIIb) ne contient pas plus de deux ribonucléotides, de préférence pas plus d'un ribonucléotide et de façon plus préférentielle aucun ribonucléotide et aucun désoxyribonucléotide.

11. Oligomère selon l'une quelconque des revendications 9 à 10, dans lequel G¹² indique un ribonucléotide, un désoxyribonucléotide ou un élément structural dans lequel W est un groupe alkyle, alcényle, alcoxy ou alcényloxy en C₁ à C₄ le cas échéant substitué par OH, de préférence un résidu 2-hydroxyéthoxy.

12. Oligomère selon l'une quelconque des revendications 9 à 11, dans lequel A¹³, A¹⁴ et/ou R^{15.1} sont choisis parmi les éléments structuraux dans lesquels W est de préférence un groupe alkyle, alcényle, alcoxy ou alcényloxy en C₁ à C₄ le cas échéant substitué par OH.

13. Oligomère selon la revendication 12, dans lequel W est choisi parmi les résidus méthoxy, 2-hydroxyéthoxy et allyloxy.

14. Oligomère selon l'une quelconque des revendication 9 à 13, dans lequel le substrat d'ARN est choisi dans le groupe comprenant l'ARNm de l'interleukine 2 humaine, l'ARNm de l'ICAM-1 humaine, l'ARNm du TGF β humain, le pré-ARNm du facteur de croissance épithéliale humain, l'ARNm de la protéine kinase C-α humaine, l'ARNm du facteur de croissance KBF-1 humain, l'ARNm de la 5-lipoxygénase humaine, l'ARN de l'interleukine 8 humaine, l'ARN de l'interleukine 5 humaine, l'ARNm du récepteur de l'interleukine 1 humaine, l'ARNm de l'interleukine 1-α humaine, l'ARNm de la 12-lipoxygénase humaine, le produit de transcription du gène de l'ADN-polymérase du cytomégalovirus humain et un produit de transcription du papillomavirus humain de type 8.

15. Oligomère selon l'une quelconque des revendication 9 à 14, dans lequel le substrat d'ARN comporte une région provenant de l'ARNm de l'interleukine 2 humaine ayant la séquence nucléotidique représentée dans la SEQ. ID. n° 1, de l'ARNm de l'ICAM-1 humaine ayant la séquence nucléotidique représentée dans la SEQ. ID. n° 3, de l'ARN de la PKC-α humaine ayant la séquence nucléotidique représentée dans la SEQ. ID. n° 9, et de l'ARNm de l'interleukine 1-α humaine ayant la séquence nucléotidique représentée dans la SEQ. ID. n° 11.

16. Oligomère selon la revendication 15, dans lequel l'oligomère contient la séquence nucléotidique représentée dans la SEQ. ID. n° 2, la SEQ. ID. n° 4, la SEQ. ID. n° 10 ou la SEQ. ID. n° 12.

17. Oligomère selon l'une quelconque des revendications 1 à 8, dans lequel le centre catalytique Z a la structure (IVa) ou (IVb) et possède une activité de coupure pour un substrat d'ARN qui contient une structure des formules (Via) ou (VIb):
5'Y^{11.1}G¹²A¹³A¹⁴R^{15.1}-S-U^{16.1}H¹⁷-3'1 (VIa)
5'-Y^{11.1}N^{λ12}G¹²A¹³A¹⁴R^{15.1}-S-U^{16.1}H¹⁷-3' (VIb)
dans laquelle :
N, H, Y, R, S, G, A, U et C représentent des ribonucléotides et
N indique G, A, U ou C,
H indique A, C ou U,
R indique A ou G,
Y indique C ou U et
S est une séquence d'ARN capable de former une structure en épingle à cheveux.

18. Oligomère selon la revendication 17, dans lequel Z a la structure (IVa) ou (IVb) et ne contient pas plus de six ribonucléotides, de préférence aucun ribonucléotide pyrimidinique.

19. Oligomère selon l'une quelconque des revendications 17 à 18, dans lequel un ou plusieurs éléments structuraux dans les positions G⁵, A⁶, N⁷, G⁸ et A⁹ sont des ribonucléotides, dans lesquels N⁷ est une base purine, en particulier A.

20. Oligomère selon l'une quelconque des revendications 17 à 19, dans lequel C³, U⁴ et N^{λ12} sont choisis parmi les éléments structuraux dans lesquels W est un groupe alkyle, alcényle, alcoxy ou alcényloxy en C₁ à C₄ le cas échéant substitué par OH, ou un groupe NH₂.

21. Oligomère selon la revendication 20, dans lequel W est choisi parmi les résidus méthoxy, 2-hydroxyéthyloxy, allyloxy, allyle et NH₂.

22. Oligomère selon l'une quelconque des revendications 17 à 21, dans lequel le substrat d'ARN est choisi dans le groupe comprenant l'ARNm de l'interleukine 6 humaine, l'ARNm de la MDR-1 humaine, l'ARN de la protéine chimiotactique des monocytes humains, l'ARNm du récepteur d'adsorption des macrophages humains de type II, l'ARNm du récepteur d'adsorption des macrophages humains de type I, l'ARNm de la protéine inflammatoire 1-α des macrophages humains, l'ARN de la p53 humaine, l'ARNm du jun-B humain, l'ARN du c-jun humain, l'ARNm de l'interféron γ humain de type II, l'ARNm du facteur de croissance des hépatocytes humains, l'ARNm de HER2 humain, l'ARNm du composant amyloïde de la maladie d'Alzheimer humaine, l'ARNm de l'interleukine 1 humaine, l'ARNm du récepteur de l'interleukine 1 humaine, la 3-hydroxy-3-méthylglutaryl-coenzyme A réductase humaine, l'ARNm de l'angiotensinogène humain, l'ARNm de l'enzyme de conversion de l'angiotensine humaine, l'ARNm de l'acylcoenzyme A : cholestérol acyltransférase humaine, l'ARNm du récepteur du PDGF humain, l'ARNm du récepteur du TNF humain, l'ARNm du TGF β humain, l'ARNm de la sous-unité p65 du NF-kappa B humain, l'ARN du c-myc humain, l'ARNm de la 12-lipoxygénase humaine, l'ARN de l'interleukine 4 humaine, l'ARNm de l'interleukine 10 humaine, l'ARNm du FGF de base humain, l'ARNm du récepteur de l'EGF humain, l'ARNm du c-myb humain, l'ARN du c-fos humain, l'ARNm du bcl-2 humain, l'ARNm du bcl-1 humain, l'ARNm de l'ICAM-1 humaine, un produit de transcription du papillomavirus humain de type 11 et des produits de transcription des papillomavirus humains de type 16 et de type 18.

23. Oligomère selon l'une quelconque des revendications 17 à 22, dans lequel le substrat d'ARN contient une région parmi l'ARNm de l'interleukine 6 humaine ayant la séquence nucléotidique représentée dans la SEQ. ID. n° 5, l'ARNm de la MDR-1 humaine ayant la séquence nucléotidique représentée dans la SEQ. ID. N °7, l'ARN de c-jun ayant la séquence nucléotidique représentée dans la SEQ. ID. n° 13, l'ARNm du récepteur de l'interleukine 1 humaine ayant la séquence nucléotidique représentée dans la SEQ. ID. n° 15 et l'ARN de l'angiotensinogène humain ayant la séquence nucléotidique représentée dans la SEQ. ID. n° 17.

24. Oligomère selon la revendication 23, dans lequel l'oligomère contient la séquence nucléotidique représentée dans la SEQ. ID. n° 6, la SEQ. ID. n° 8, la SEQ. ID. n° 14, la SEQ. ID. n° 16 et la SEQ. ID. n° 18.

25. Oligomère selon l'une quelconque des revendications 1 à 24, dans lequel ledit oligomère est lié à un groupe prosthétique.

26. Composition pharmaceutique contenant un ou plusieurs oligomères chimériques selon l'une quelconque des revendications 1 à 25 comme substance active et le cas échéant des substances auxiliaires, des additifs et des véhicules pharmaceutiquement acceptables.

27. Utilisation d'un oligomère chimérique selon l'une quelconque des revendications 1 à 25 ou d'une composition pharmaceutique selon la revendication 26 pour produire un agent pour inactiver spécifiquement l'expression de gènes dans les eucaryotes, les procaryotes et les virus.

28. Utilisation pour préparer un médicament selon la revendication 27 pour inactiver spécifiquement l'expression de gènes humains dans une cellule, pour inactiver spécifiquement l'expression de gènes tumoraux, pour inactiver spécifiquement l'expression de gènes viraux ou de molécules d'ARN dans une cellule, ou pour inactiver spécifiquement l'expression de gènes végétaux.

29. Utilisation selon l'une quelconque des revendications 27 à 28, dans laquelle la substance active est administrée à une concentration de 0,01 à 10 000 µg par kg de masse corporelle.

30. Utilisation selon l'une quelconque des revendications 27 à 29, dans laquelle la substance active est administrée par injection, inhalation, sous forme de spray, par voie orale, locale ou rectale.

31. Procédé pour inactiver spécifiquement in vitro l'expression de gènes,
dans lequel un oligomère chimérique selon l'une quelconque des revendications 1 à 25 est introduit dans une cellule en une concentration active afin que l'oligomère coupe spécifiquement une molécule d'ARN prédéterminée présente dans la cellule.

32. Procédé selon la revendication 31, dans lequel la coupure se produit de manière catalytique.

33. Utilisation d'un oligomère chimérique selon l'une quelconque des revendications 1 à 25 pour fabriquer un médicament destiné à inactiver spécifiquement l'expression de gènes, dans laquelle ledit oligomère chimérique est introduit dans une cellule en une concentration active afin que l'oligomère coupe spécifiquement une molécule d'ARN prédéterminée présente dans la cellule.

34. Utilisation selon la revendication 33, dans laquelle la coupure se produit de manière catalytique.

35. Utilisation d'un oligomère chimérique selon l'une quelconque des revendications 1 à 25 comme enzyme de restriction d'ARN, comme agent de diagnostic, ou pour identifier la fonction de gènes inconnus.

36. Kit pour la coupure de restriction de molécules d'ARN, dans lequel ledit kit contient un oligomère chimérique selon l'une quelconque des revendications 1 à 25 ainsi que des substances tampon adaptées.

37. Procédé de préparation d'une composition pour soulager le psoriasis, comprenant la formulation d'une quantité efficace d'un oligomère choisi dans le groupe comprenant des oligomères selon les revendications 1 à 13 et 17 à 21, dans lequel le substrat d'ARN est un ARN d'IL-2 ou d'ICAM-1,

38. Procédé de préparation d'une composition pour soulager le rhume courant, comprenant la formulation d'une quantité efficace d'un oligomère choisi dans le groupe comprenant des oligomères selon les revendications 1 à 13 et 17 à 21, dans lequel le substrat d'ARN est un ARN d'ICAM-1.

39. Procédé de préparation d'une composition pour soulager le rejet d'une greffe, comprenant la formulation d'une quantité efficace d'un oligomère choisi dans le groupe comprenant des oligomères selon les revendications 1 à 13 et 17 à 21, dans lequel le substrat d'ARN est un ARN d'ICAM-1.

40. Procédé de préparation d'une composition pour soulager la sarcomatose multiple hémorragique de Kaposi, comprenant la formulation d'une quantité efficace d'un oligomère choisi dans le groupe comprenant des oligomères selon les revendications 1 à 13 et 17 à 21, dans lequel le substrat d'ARN est un ARN de IL-6.

41. Procédé de préparation d'une composition pour traiter le cancer, comprenant la formulation d'une quantité efficace d'un oligomère choisi dans le groupe comprenant des oligomères selon les revendications 1 à 13 et 17 à 21, dans lequel le substrat d'ARN est un ARN de PKC-α.

42. Procédé de préparation d'une composition pour traiter l'hypertension, comprenant la formulation d'une quantité efficace d'un oligomère choisi dans le groupe comprenant des oligomères selon les revendications 1 à 13 et 17 à 21, dans lequel le substrat d'ARN est un ARN d'angiotensinogène.


### SEQUENCE LISTING

(1) GENERAL INFORMATION:
   (i) APPLICANT:
      (A) NAME: VimRx Holdings, Ltd.
      (B) STREET: 2751 Centerville Road, Suite 210
      (C) CITY: Little Falls II, Wilmington
      (E) COUNTRY: USA
      (F) POSTAL CODE (ZIP): Delaware 19808
   (ii) TITLE OF INVENTION: Chimeric oligomers having an RNA-cleavage activity
   (iii) NUMBER OF SEQUENCES: 18
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: DE 195 42 404.2
      (B) FILING DATE: 14-NOV-1995
(2) INFORMATION FOR SEQ ID NO: 1:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO: 1:
   AACAACAUUC AUGUGUGAAU AUGCUGAUGA GACAGCAACT 40
(2) INFORMATION FOR SEQ ID NO: 2:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 2:
   GUUGCUGUCG AAAUGUUGUU T 21
(2) INFORMATION FOR SEQ ID NO: 3:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO: 3:
   GACAUGAUUG AUGGAUGUUA AAGUCUAGCC UGAUGAGAGG GGT 43
(2) INFORMATION FOR SEQ ID NO: 4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO: 4:
   CCCCUCGAAA UCAUGUCT 18
(2) INFORMATION FOR SEQ ID NO: 5:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO: 5:
   UACCACUUGA AACAUUUUAU GUAUUAGUUU UT 32
(2) INFORMATION FOR SEQ ID NO: 6:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 6:
   AAAACUAACU GAUGAAAGUG GUAT 24
(2) INFORMATION FOR SEQ ID NO: 7:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO: 7:
   GCAGCUUAUG AAAUCUUCAA GAUAAUUGAU T 31
(2) INFORMATION FOR SEQ ID NO: 8:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO: 8:
   AUCAAUCUGA UGAAUAAGCU GCT 23
(2) INFORMATION FOR SEQ ID NO: 9:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO: 9:
   GAUCCCUUUC CUUUGGAGUU UCGGAGCUGA UGAAGAUGCC T 41
(2) INFORMATION FOR SEQ ID NO: 10:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO: 10:
   GGCAUCUGAA AAAGGGAUT 19
(2) INFORMATION FOR SEQ ID NO: 11:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 11:
   UGUUUGCCUU CUACUUUUAA GUUGCUGAUG AACUCUUAT 39
(2) INFORMATION FOR SEQ ID NO: 12:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO: 12:
   UAAGAGUGAA AGGCAAACT 19
(2) INFORMATION FOR SEQ ID NO: 13:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO: 13:
   UAGGUGGAGU UGAAAGAGUU AAGAAUGCUC GAUAAAAUCT 40
(2) INFORMATION FOR SEQ ID NO: 14:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO: 14:
   UUUUAUCCUG AUGAAACUCC AT 22
(2) INFORMATION FOR SEQ ID NO: 15:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO: 15:
   AAGAAGAAUA UGAAAGUGUU ACUCAGACUU AUUT 34
(2) INFORMATION FOR SEQ ID NO: 16:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO: 16:
   UAAGUCUCUG AUGAAUAUUC UT 22
(2) INFORMATION FOR SEQ ID NO: 17:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 17:
   AAUACGUGAA AGAUGCAAGC ACCUGAAUUU CUGUUUGAT                                  39
(2) INFORMATION FOR SEQ ID NO: 18:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO: 18:
   UCAAACACUG AUGAACGUAU UT                                                    22